(19) Europäisches Patentamt / European Patent Office / Office européen des brevets

(11) **EP 2 908 788 B1**

(12) **EUROPÄISCHE PATENTSCHRIFT**

(45) Veröffentlichungstag und Bekanntmachung des Hinweises auf die Patenterteilung:
**25.03.2020   Patentblatt 2020/13**

(21) Anmeldenummer: **13773672.4**

(22) Anmeldetag: **30.09.2013**

(51) Int Cl.:
**A61B 3/00** (2006.01)       **A61F 9/008** (2006.01)
**A61F 9/007** (2006.01)       **A61F 9/009** (2006.01)

(86) Internationale Anmeldenummer:
**PCT/EP2013/070345**

(87) Internationale Veröffentlichungsnummer:
**WO 2014/060206 (24.04.2014 Gazette 2014/17)**

(54) **VORRICHTUNG ZUR BESTRAHLUNG DES AUGES**

DEVICE FOR IRRADIATING THE EYE

DISPOSITIF D'EXPOSITION DE L' OEIL À UN RAYONNEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.10.2012   AT 11222012**

(43) Veröffentlichungstag der Anmeldung:
**26.08.2015   Patentblatt 2015/35**

(73) Patentinhaber: **Daxer, Albert**
**4073 Wilhering (AT)**

(72) Erfinder: **Daxer, Albert**
**4073 Wilhering (AT)**

(74) Vertreter: **KLIMENT & HENHAPEL**
**Patentanwälte OG**
**Gonzagagasse 15/2**
**1010 Wien (AT)**

(56) Entgegenhaltungen:
**EP-A1- 2 327 383       WO-A1-2010/022745**
**WO-A2-2013/059837     US-A1- 2009 209 954**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

GEBIET DER ERFINDUNG

Die Erfindung betrifft eine Vorrichtung und ein Verfahren zur Bestrahlung des - vorzugsweise menschlichen - Auges, insbesondere der Hornhaut. Die erfindungsgemäße Vorrichtung ist darauf gerichtet, Veränderungen in der Struktur des Hornhautstromas herbeizuführen, die krankhaften Veränderungen entgegenwirken. So ist beispielsweise der Keratokonus eine Erkrankung der Hornhaut, die durch fortschreitenden Umbau der Struktur des Hornhautstromas zu einer zunehmenden Schwächung der mechanischen Stabilität des Gewebes führt. Dies bewirkt wiederum eine Veränderung der Hornhautgeometrie, die zu einer erheblichen Sehverschlechterung bis hin zu einer hornhautbasierten Erblindung führen kann. Ähnliches gilt für Keratoglobus, pellucid marginal degeneration (PMD), post-LASIK Keratektasie, progredienter Myopie und anderen Augenleiden.

STAND DER TECHNIK

Als Stand der Technik gilt die korneale Quervernetzung der Kollagenfibrillen der Hornhaut durch die Einbringung von Riboflavin in das Hornhautstroma kombiniert mit einer UV-A Bestrahlung. Dadurch soll die Stabilität der Hornhaut erhöht und ein Fortschreiten der Erkrankung verhindert werden. Auch kann durch die Quervernetzung die Geometrie der Hornhaut verändert und dadurch eine refraktive Korrektur am Auge vorgenommen werden.

So beschreibt die WO 2012/047307 A1 eine Vorrichtung zur Bestrahlung der Hornhaut zur Quervernetzung der Kollagenfibrillen des Gewebes, nachdem in die Hornhaut Riboflavin als Agens (Photosensitizer) eingebracht wurde. Der Nachteil eines solchen Systems ist, dass das System bei Augenbewegungen eine fehlerhafte Bestrahlung des Auges vornehmen kann.

EP 1561440 A1, US2009/209954 und WO 2012/127330 A1 beschreiben jeweils eine Vorrichtung zur Verformung und Verfestigung der Hornhaut durch Aufsetzen eines Formkörpers auf die Hornhaut, durch welchen hindurch die Bestrahlung der Hornhaut als auch die Fixierung des Formkörpers bzw. der Bestrahlungseinrichtung an der Hornhaut erfolgt. Hintergrund dieser Erfindung ist die Tatsache, dass die Brechkraft (Dioptrien) des Auges sehr stark vom Krümmungsradius der Vorderfläche (Oberfläche) der Hornhaut abhängt. Durch eine neue Formgebung der Hornhautoberfläche mit einem geeigneten Formkörper sollte es möglich sein, so die Hypothese der beiden Druckschriften, die Brechkraft des Auges durch diese Vorrichtung definiert zu verändern. Ob diese Hypothese richtig ist, soll hier nicht diskutiert werden. Ein wesentlicher Nachteil dieser beiden Systeme ist jedenfalls, dass die Ansaugung an der Hornhaut eben dort erfolgt, wo auch die Bestrahlung erfolgt, nämlich flächig auf der Hornhautoberfläche durch den besagten Formkörper. Eine Ansaugung an der Hornhautoberfläche, wie in den beiden Druckschriften des Standes der Technik beschrieben, kann leicht zu einer Verletzung der Hornhautoberfläche im Sinne einer Erosio corneae führen, was für den Patienten erhebliche, tagelang andauernde Schmerzen verursachen kann. Zwar ist das klassische Verfahren zur Bestrahlung der Hornhaut, wie z.B. in WO 2012/047307 A1 beschrieben, selbst mit einer Erosio corneae und erheblichen Schmerzen verbunden, doch gibt es auch neuere Ansätze zur Behandlung des Keratokonus, wo keine Erosio corneae mehr notwendig ist und wo daher die Behandlung schmerzfrei ablaufen könnte (A. Daxer et al. Corneal Crosslinking and Visual Rehabilitation in Keratoconus in One Session Without Epithelial Debridement: New Technique. CORNEA 2010;29:1176-1179). Speziell für solche Anwendungen, wenn das Epithel bei der Behandlung grundsätzlich erhalten werden kann, weisen die beiden Vorrichtungen aus der EP 1561440 A1 und WO 2012/127330 A1 aufgrund des Ansaugens an der zu bestrahlenden Hornhautfläche einen erheblichen Nachteil auf.

DARSTELLUNG DER ERFINDUNG

Es ist daher eine Aufgabe der vorliegenden Erfindung, eine Vorrichtung zur Bestrahlung der Hornhaut des Auges zur Verfügung zu stellen, welche eine Quervernetzung der Kollagenfibrillen unter Überwindung der Nachteile des Standes der Technik ermöglichen. Es werden verschiedene Ausführungsformen der Vorrichtung und dazugehörig verschiedene Verfahren der Behandlung vorgestellt. Jede Ausführungsform und das dazugehörige Verfahren sind auch als Ausgangspunkt weiterer Ausführungsformen zu verstehen, indem ein oder mehrere Teile oder Elemente der vorgestellten Ausführungsformen wieder so mit einem oder mehreren Teilen und Elementen anderer Ausführungsformen kombiniert werden können, dass neue Ausführungsformen entstehen.

Die Aufgabe wird gelöst durch eine Vorrichtung gemäß Anspruch 1, welche zumindest die folgenden Elemente umfasst:

- einen Ringkörper, der eine konzentrisch um eine Längsachse der Vorrichtung ausgeführte Auflagefläche zum Zwecke der Befestigung der Vorrichtung am Auge aufweist,
- einen Bestrahlungskanal zur Bestrahlung der Hornhaut, der sich innerhalb des Ringkörpers befindet,

- eine Lichtquelle, die im betriebsbereiten Zustand der Vorrichtung zum Abgeben von Licht in den Bestrahlungskanal innerhalb des Ringkörpers angebracht ist,
- wobei die Auflagefläche zur Befestigung der Vorrichtung außerhalb des Bestrahlungskanals angeordnet ist.

Dadurch, dass die Auflagefläche außerhalb des Bestrahlungskanals vorgesehen ist (nämlich außerhalb in Normalrichtung zur Längsachse der Vorrichtung bzw. des Ringkörpers gesehen), liegt die Vorrichtung im Betriebszustand nur außerhalb der bestrahlten Fläche am Auge auf, die bestrahlte Fläche selbst wird nicht durch Auflageflächen der Vorrichtung zusätzlich belastet. Die erfindungsgemäße Vorrichtung weist also innerhalb des Bestrahlungskanals keine Einbauten zur Befestigung am Auge auf, die im betriebsbereiten Zustand der Vorrichtung, wenn diese am Auge befestigt ist, das Auge berühren. Insbesondere kann der Bestrahlungskanal an der - im Betriebszustand der Vorrichtung - dem Auge zugewandten Seite (= an der der Lichtquelle abgewandten Seite) offen sein.

Die Auflagefläche bzw. ein Durchmesser der Auflagefläche bzw. ein Durchmesser des Ringkörpers bzw. ein Durchmesser des Saugrings ist dabei so dimensioniert, dass die Befestigung am Auge vorzugsweise außerhalb der Hornhaut, bzw. außerhalb des Limbus bzw. im Bereich der Bindehaut stattfindet.

Die Form des Bestrahlungskanals kann grundsätzlich beliebig sein, ist jedoch zumindest abschnittsweise entlang der Längsachse, bevorzugt als Innenraum (Hohlraum) eines Hohlzylinders, ausgeführt und von der Innenwand des Ringkörpers begrenzt. Der Durchmesser der Austrittsöffnung, gemessen senkrecht zur Längsachse, darf dabei den Innendurchmesser (kleinster Durchmesser) der bevorzugt konzentrisch um die Längsachse angeordneten Ansaugfläche nicht überschreiten. In einer bevorzugten Ausführungsvariante soll der kleinste Abstand der Begrenzung der Austrittsöffnung (Austrittsfläche) von der Längsachse den kleinsten Abstand der inneren Auflagekante bzw. den kleinsten Abstand der Ansaugfläche von der Längsachse nicht überschreiten. Der Durchmesser des Bestrahlungskanals, gemessen senkrecht zur Längsachse, soll zumindest abschnittsweise bevorzugt zwischen 0,5 mm und 20 mm liegen und in einer besonders bevorzugten Ausführungsvariante zwischen 8 mm und 18 mm, z.B. ca. 15 mm sein. Der Durchmesser der Austrittsöffnung (Austrittsfläche, Bestrahlungsfläche) aus dem Bestrahlungskanal, gemessen auf der Höhe der Stirnfläche des Ringkörpers bzw. der inneren Auflagekante entlang der Längsachse sollte möglichst 12 mm nicht überschreiten und den Durchmesser des Verlaufs der inneren Auflagekante bzw. den inneren Durchmesser der Ansaugfläche von möglichst mehr als 10 mm nicht überschreiten, sodass der Vorrichtungsteil zur Befestigung am Auge jedenfalls außerhalb der bestrahlten Fläche liegt bzw. diesen nach außen hin, gemessen senkrecht zur Längsachse, begrenzt. In einer besonderen Ausführungsform können zwar Einbauten im Bestrahlungskanal das Auge bzw. die Hornhaut berühren, aber ohne an der Befestigung der Vorrichtung am Auge (z.B. Ansaugung) beteiligt zu sein.

Als Lichtquelle im Sinne der gegenständlichen Erfindung ist jede Strahlungsquelle anzusehen, die elektromagnetische Strahlung im Bereich von Ultraviolettstrahlung bis Infrarotstrahlung aussenden kann, insbesondere jedoch Ultraviolettstrahlung. Die Lichtquelle wird im betriebsbereiten Zustand in der Regel in Längsrichtung nicht über den Ringkörper hinausragen.

Der Bestrahlungskanal, der sich innerhalb des Ringkörpers befindet, könnte in besonders einfacher Weise durch die Innenwand des Ringkörpers gebildet werden.

Die erfindungsgemäße Vorrichtung ist im Wesentlichen als Ringkörper ausgeführt, der im Wesentlichen (mehr oder weniger) konzentrisch um die Längsachse angeordnet ist. In einem besonders einfachen Fall ist der Ringkörper als Hohlzylinder ohne Deckflächen ausgebildet, also als Zylindermantel. Die Längsachse der Vorrichtung entspricht dann der Zylinderachse. Ein Ringkörper im Sinne der gegenständlichen Erfindung ist aber jeder Körper, der über einen geschlossenen Mantel verfügt, wobei der Mantel eine Längsachse umgibt. Innerhalb des Mantels ist zumindest ein Hohlraum, der in Längsrichtung an einer Seite, insbesondere an beiden Seiten, offen ist.

Der Ringkörper kann über einen konzentrischen Saugring verfügen, der etwa an einem Ende des Hohlzylinders angeordnet ist, und über einen Aufsatz, der mit dem Saugring in Verbindung steht (insbesondere mechanisch starr) und eine Aufnahme für die funktionellen Komponenten, wie z.B. Lichtquelle, Energiequelle bzw. Energiezufuhr, Elektronik (Steuerelektronik), aufweist. Der Aufsatz kann einteilig mit dem Ringkörper ausgeführt sein, insbesondere durch den anderen, dem Saugring abgewandten Endbereich des Ringkörpers. Der Saugring kann an der Stirnfläche des Ringkörpers, welche die Auflagefläche bildet, angeordnet sein.

Eine Ausführungsform der Erfindung sieht vor, dass die Lichtquelle in fester, insbesondere nicht lösbarer, mechanischer Verbindung mit einer Steuerelektronik zur Steuerung der Lichtquelle steht und die Steuerelektronik im betriebsbereiten Zustand der Vorrichtung ebenfalls innerhalb des vom Ringkörper definierten Innenraums der Vorrichtung angebracht ist. Der Vorteil dieser Ausführungsform liegt darin, dass eine kompakte Ausführung der Vorrichtung sowie gleichzeitig ein gewünschter Verlauf der Bestrahlung erreicht werden kann.

In einer weiteren Ausführungsform stehen die funktionellen Komponenten Lichtquelle, Energiequelle bzw. Energiezufuhr zum Betrieb der Bestrahlungsquelle und Elektronik (Steuerelektronik) untereinander in fester - insbesondere nicht lösbarer -mechanischer Verbindung, was ebenfalls zur kompakten Ausführung der Vorrichtung beiträgt, insbesondere, wenn auch die Energiequelle innerhalb des Ringkörpers angeordnet ist. Die Lichtquelle, Energiequelle und Steuerelektronik könnte z.B. in der Aufnahme fix eingebaut und fest mit dem Aufsatz bzw. dem Ringkörper verbunden sein.

Die Aufnahme stellt dabei einen - zumindest seitlich (normal zur Längsachse) - abgeschlossenen Innenraum der Vorrichtung dar. Die funktionellen Komponenten sind dann nicht auswechselbar. D.h. eine Aufnahme im eigentlichen Sinne ist nicht vorhanden, da die funktionellen Komponenten mit dem Ringkörper in fester, nicht veränderbarer räumlicher Beziehung sind.

Vorzugsweise ist die gesamte Vorrichtung steril als medizinisches Einmalprodukt ausgeführt. Die gesamte Vorrichtung ist einschließlich der eingebauten funktionellen Komponenten daher so ausgeführt, dass sie entweder mittels Ethylenoxid oder Gamma Strahlung sterilisierbar ist. Der Ringkörper mit Saugring und die gesamten Außenteile der Vorrichtung können damit als Spritzgussteil aus biokompatiblen Material, z.B. PMMA oder einem anderen geeigneten Kunststoff, ausgeführt werden. Grundsätzlich kann die Vorrichtung aus jedem beliebigen geeigneten Material ausgeführt sein.

Der Saugring verfügt über einen Anschluss, mit dem die Ansaugfläche an eine Saugpumpe angeschlossen werden kann, sodass der Ringkörper durch den erzeugten Unterdruck am Auge befestigt wird.

Um eine möglichst kompakte Ausführung der Vorrichtung zu erzielen, die nur teilweise ein Wegwerfprodukt ist, kann vorgesehen sein, dass Lichtquelle, Steuerelektronik und Energiequelle von einem gemeinsamen Gehäuse umschlossen sind. Das Gehäuse kann dann in den Ringkörper eingesetzt und auch wieder entnommen werden, sodass Ringkörper und Gehäuse aufgrund der lösbaren Verbindung getrennt werden können und einer der beiden Teile wiederverwendet werden kann.

Entsprechend besteht eine Variante der Erfindung darin, dass der Ringkörper eine Aufnahme für ein Gehäuse aufweist, in welchem zumindest die Lichtquelle, vorzugsweise aber weitere funktionelle Komponenten - insbesondere (mechanisch) lösbar - angebracht ist.

Die Aufnahme kann integraler Bestandteil des Ringkörpers und einteilig mit ihm ausgeführt sein, sie könnte aber auch als separater Teil gefertigt und dann fest oder lösbar mit dem Ringkörper verbunden werden.

Um im Falle der Ausführung mit Gehäuse im betriebsbereiten Zustand der Vorrichtung einen definierten Abstand zwischen Lichtquelle und Auge sicherzustellen, kann vorgesehen sein, dass die Aufnahme eine Anschlagbegrenzung aufweist, sodass ein Gehäuse nur bis zu einer durch die Anschlagbegrenzung direkt oder indirekt definierten Tiefe in den Ringkörper eingebracht werden kann. Damit das Gehäuse nicht - von der Anschlagbegrenzung weg - aus dem Ringkörper fallen kann, können zusätzliche Haltevorrichtungen, wie Rasten oder Klemmvorrichtungen, vorgesehen sein.

Um eine Übertragung von Keimen von einem möglicher Weise nicht sterilen Gehäuse auf den Ringkörper und in weiterer Folge auf das Auge zu vermeiden, kann vorgesehen sein, dass die Vorrichtung über eine Gehäusehülle verfügt, die eine anschlagbegrenzte Aufnahme für das Gehäuse aufweist, wobei die Gehäusehülle selbst in die Aufnahme für ein Gehäuse im Ringkörper, vorzugsweise anschlagbegrenzt, eingebracht werden kann. Dabei sollte im betriebsbereiten Zustand die Gehäusehülle das Gehäuse am Boden und seitlich vollständig und dicht umgeben und an dessen Oberseite am besten überragen.

Eine Ausführungsform der Erfindung sieht vor, dass zumindest die Lichtquelle von einem Gehäuse umschlossen ist, welches einen Boden besitzt, der im betriebsbereiten Zustand dem Bestrahlungskanal zugewandt ist und zumindest teilweise für das Bestrahlungslicht, welches von der Lichtquelle erzeugt wird, transparent ist. Dadurch wird die Lichtquelle vollständig von Keimen abgeschirmt, und es muss gegebenenfalls nur das Gehäuse, nicht jedoch die Lichtquelle sterilisiert werden.

Im Falle einer Gehäusehülle kann entsprechend vorgesehen sein, dass die Gehäusehülle einen Boden besitzt, der zumindest teilweise für das Bestrahlungslicht, welches von der Lichtquelle erzeugt wird, transparent ist.

Mit einer Steuerelektronik für die Lichtquelle können unterschiedliche Einstellungen vorgenommen werden. Insbesondere kann eine Steuerelektronik für die Lichtquelle vorgesehen sein, mit welcher die Bestrahlungsleistung so einstellbar ist, dass sie während der Bestrahlung der Hornhaut abnimmt.

Die erfindungsgemäße Vorrichtung kann über eine Kühlvorrichtung (z.B. Ventilator, Kühlkörper, Kühlflüssigkeit, etc.) zur Abführung der von der Lichtquelle erzeugten Verlustwärme verfügen. Dadurch sind die übrigen Bestandteile der Vorrichtung einer geringeren Wärme ausgesetzt und können aus Materialien mit geringerer Temperaturfestigkeit hergestellt werden.

Ein Verfahren zur Bestrahlung der Hornhaut eines Auges unter Verwendung einer erfindungsgemäßen Vorrichtung sieht vor, dass die Vorrichtung am Auge befestigt wird und die Bestrahlungsleistung während der Bestrahlung der Hornhaut verändert wird. Um die mit zunehmender Bestrahlungsdauer steigende Erwärmung der Vorrichtung zu verringern, kann vorgesehen werden, dass die Bestrahlungsleistung während der Bestrahlung der Hornhaut abnimmt. Die Erfindung wird in den Ansprüchen definiert.

KURZE BESCHREIBUNG DER FIGUREN

Zur weiteren Erläuterung der Erfindung wird im nachfolgenden Teil der Beschreibung auf die Figuren Bezug genommen, aus denen weitere vorteilhafte Ausgestaltungen, Einzelheiten und Weiterbildungen der Erfindung zu entnehmen sind. Es zeigen:

Fig. 1    einen Querschnitt durch ein Auge mit schematischer Lichtquelle,

Fig. 2    einen Längsschnitt durch eine erfindungsgemäße, am Auge befestigte Vorrichtung,

Fig. 3    die Vorrichtung aus Fig. 2 mit Energiequelle,

Fig. 4    eine alternative Ausführungsform der Vorrichtung aus Fig. 2 mit Gehäuse und Gehäusehülle,

Fig. 5    einen Kühlkörper für eine erfindungsgemäße Vorrichtung,

Fig. 6    ein Diagramm über die in Abhängigkeit von der Zeit mit der erfindungsgemäßen Vorrichtung ins Auge eingebrachte Leistung,

Fig. 7    ein weiteres Diagramm über die in Abhängigkeit von der Zeit mit der erfindungsgemäßen Vorrichtung ins Auge eingebrachte Leistung.

## WEGE ZUR AUSFÜHRUNG DER ERFINDUNG

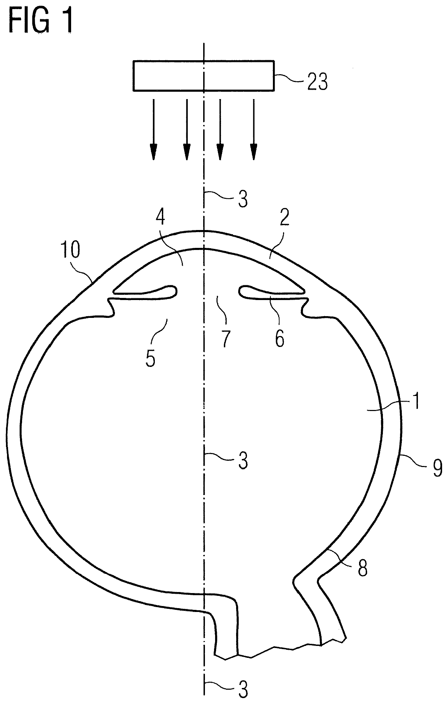

Der prinzipielle Aufbau des Auges sowie das Prinzip der erfindungsgemäßen Bestrahlung sind in Fig. 1 schematisch dargestellt. Das Auge 1 ist im Wesentlichen eine Hohlkugel, die vorne von der Hornhaut 2 begrenzt wird. Die Begriffe vorne und hinten sind in der Anatomie des Menschen eindeutig festgelegt. Als Symmetrieachse 3 des Auges gilt die optische oder anatomische Achse des Auges, um welche das Auge 1 funktionell oder anatomisch grob rotationssymmetrisch angeordnet ist. Die anatomische und die optische Achse fallen nicht notwendigerweise zusammen. In Bezug auf Details zum Verhältnis zwischen optischer und anatomischer Achse wird auf die einschlägige Fachliteratur verwiesen. Die vordere 4 und die hintere 5 Augenkammer werden durch die Iris 6 getrennt. Die zentrale Öffnung der Iris bildet die Pupille 7, durch welche das Licht in die hintere Augenkammer 5 und damit auf die Netzhaut 8 gelangen kann. Anschließend an die Hornhaut bildet die Sklera 9 die äußere Wand des Auges 1. Die Sklera 9 ist zumindest im vorderen Teil des Auges außen durch die Bindehaut bedeckt. Den Übergang zwischen Hornhaut 2 und Sklera 9 bezeichnet man als Limbus 10. Die Hornhaut 2 besitzt eine Vorderfläche (Oberfläche) welche die äußere Oberfläche des Auges 1 nach vorne (außen) darstellt und eine Hinterfläche, welche die Vorderkammer (vordere Augenkammer) 4 nach vorne begrenzt. Die Symmetrieachse des Auges 1 sollte möglichst mit der Symmetrieachse bzw. Längsachse 3 der Vorrichtung übereinstimmen. D.h. die Vorrichtung sollte möglichst so am Auge 1 angebracht bzw. ausgerichtet sein, dass die Längsachse 3 der Vorrichtung an der optischen Achse oder anatomischen Achse des Auges 1 bzw. einer aus diesen beiden Achsen des Auges abgeleiteten Achse ausgerichtet sein. Als von der optischen oder anatomischen Achse abgeleitete Achse des Auges gilt z.B. eine Achse die durch einen Punkt auf der Hornhautoberfläche verläuft, der auf der geraden oder gekrümmten Verbindungsstrecke zwischen dem Durchtrittspunkt der optischen Achse durch die Hornhaut (z.B. 1. Purkinje Reflex) und jenem der anatomischen Achse (z.B. Pupillenmittelpunkt) verläuft. Bei der erfindungsgemäßen Bestrahlung wird Licht aus einer Lichtquelle 23 vorzugsweise parallel zur Symmetrieachse 3 des Auges 1 in die Hornhaut 2 eingebracht. In einer besonderen Ausführungsform kann durch Kollimatoren, die in die Vorrichtung (Ringkörper bzw. Gehäuse) eingebracht sind, eine Ausrichtung zumindest eines Teiles des Bestrahlungslichtes erreicht werden (Bestrahlungsrichtung), die nicht notwendigerweise parallel zur Längsachse 3 zu sein braucht und mit der Richtung der Längsachse einen von Null verschiedenen Winkel (vorzugsweise zwischen 0° und 90°) einschließen kann.

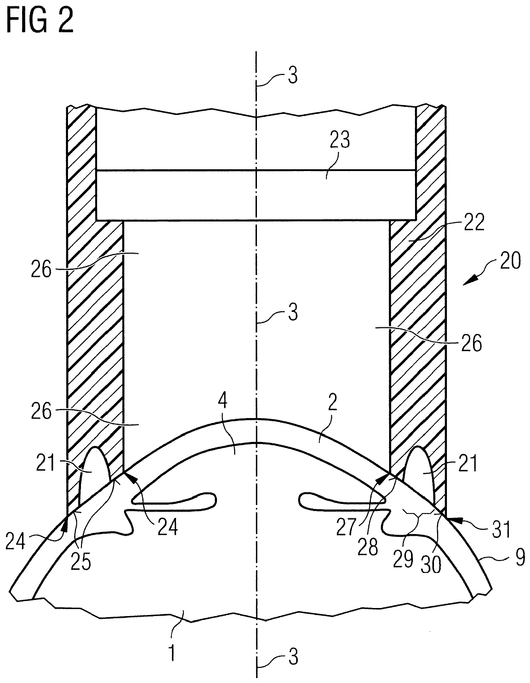

Eine bevorzugte Ausführungsform der erfindungsgemäßen Vorrichtung ist in Fig. 2 dargestellt. Sie verfügt über einen Ringkörper 20, der hier in Wesentlichen als Hohlzylindermantel ausgebildet ist. Der Ringkörper 20 weist an einem Ende einen Saugring 21 zum Ansaugen der Vorrichtung an das Auge 1 während der Behandlung auf. Der Saugring 21 ist als ringförmige Vertiefung in der Stirnfläche 25 des Ringkörpers 20 ausgebildet, innerhalb und außerhalb des Saugrings 21 verbleibt von der Stirnfläche 25 eine innere Auflagefläche 28 und eine äußere Auflagefläche 30. Die Fläche zwischen innerer und äußerer Auflagefläche 28, 30, die durch gedachte Fortsetzung von innerer und äußerer Auflagefläche 28, 30 entsteht, bildet die sogenannte Ansaugfläche 29. Die Stirnfläche 25 des Ringkörpers 20, mit welcher der Ringkörper 20 im Betriebszustand auf dem Auge 1 aufliegt, ist innen und außen von Auflagekanten 24 begrenzt, an der Innenseite von einer inneren Auflagekante 27 und an der Außenseite von einer äußeren Auflagekante 31.

In dieser besonderen Ausführungsform weist der Ringkörper 20 eine Aufnahme 22 für eine Lichtquelle 23 zur Bestrahlung der Hornhaut 2 oder Sklera 9 auf. Dabei ist der Saugring 21 bzw. der Ringkörper 20 weitgehend konzentrisch um eine Längsachse 3 der Vorrichtung ausgeführt. Konzentrisch bedeutet, dass der Saugring 21 oder eine äquivalente Struktur, wie die Auflagekante 24 bzw. Stirnfläche 25 (bzw. die innere Auflagekante 27, die äußere Auflagekante 31, die innere und äußere Auflagefläche 28, 30) in einer vorzugsweise geschlossenen, nicht notwendigerweise rotationssymmetrischen Geometrie um die Längsachse 3 bzw. einem Mittelpunkt dieser Struktur angeordnet ist. Im Grunde kann der Saugring 21 bzw. die Auflagekante 24 bzw. Stirnfläche 25 jede beliebige (geschlossene) Verlaufsform um ein Zentrum, vorzugsweise die Längsachse 3, aufweisen. Dabei braucht, in einer besonderen Ausführungsform, die Längsachse 3 selbst nicht gerade verlaufen, sondern kann auch gekrümmt oder abgewinkelt o.ä. entlang deren Verlaufes sein. In einer besonderen Ausführungsform ist die Auflagekante 24 entlang des Umfanges um das Zentrum bzw. die Längsachse 3 nicht geschlossen. In diesem Fall kann es sich um einen segmenthaften Verlauf handeln.

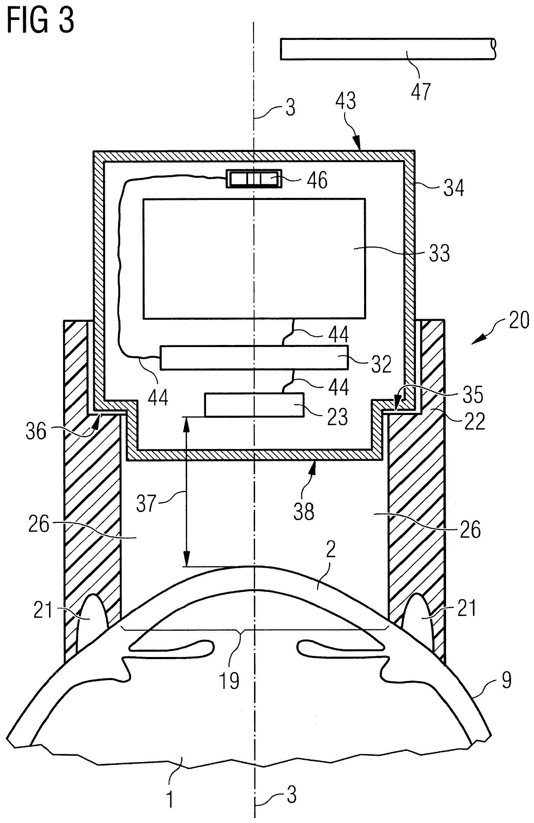

Der Saugring 21 ist nach innen, in Richtung zur Längsachse 3 hin, durch eine vorzugsweise konzentrisch zur Längsachse 3 geformten innere Auflagekante 27 bzw. innere Auflagefläche 28, die mit dem Auge 1 im betriebsbereiten Zustand in Kontakt steht bzw. das Auge 1 oder die Bindehaut oder die periphere Hornhaut oder den Limbus 10 berührt, von einem Bestrahlungsvolumen bzw. einem Bestrahlungskanal 26 bzw. einer Bestrahlungsöffnung bzw. einem Bestrahlungsbereich getrennt. Der Austritt des Lichtes aus der Vorrichtung auf die Hornhaut 2 erfolgt daher nicht durch den Ringkörper 20 oder den Saugring 21 hindurch, sondern in der zentralen Fläche (Ausnehmung der Vorrichtung) entlang und um die Längsachse 3 der Vorrichtung, worauf nach außen hin, also von der Längsachse 3 weg, mittelbar oder unmittelbar der Saugring 21, der nicht von dem Bestrahlungslicht durchdrungen wird, anschließt. Mit anderen Worten gesagt umschließt der Saugring 21 bzw. die innere Auflagekante 27 bzw. die innere Auflagefläche 28 bzw. die Aufnahme der Vorrichtung einen um die Längsachse 3 angeordneten zentralen Bereich der Vorrichtung bzw. begrenzt diesen nach außen hin, wobei in bzw. durch zumindest einen Längsabschnitt dieses zentralen Bereichs, nämlich dem Bestrahlungskanal 26, die Bestrahlung der Hornhaut 2 erfolgt. Die Bestrahlungsöffnung (Austrittsöffnung 19 für den Lichtaustritt, siehe Fig. 3) bzw. die Bestrahlungsfläche des Bestrahlungskanals 26 einerseits und der Saugring 21 bzw. die Ansaugfläche 29 andererseits sind nicht deckungsgleich und in einer besonderen Ausführungsform auch nicht überlappend. Genauer gesagt, die Bestrahlungsfläche auf der Hornhaut 2, bzw. deren Projektion auf die Hornhaut 2 in Richtung der Längsachse 3, und die Ansaugfläche 29 der Vorrichtung auf dem Auge 1 sind nicht deckungsgleich oder nicht überlappend.

Im gegenständlichen Ausführungsbeispiel ist der Bestrahlungskanal 26 zylindrisch und konzentrisch um die Längsachse 3 ausgebildet, der Saugring 21 ist kreisringförmig, ebenso die Auflageflächen 28, 30, die Auflagekanten 24, 27, 31 sind kreisförmig. In einer besonderen Ausführungsform kann die Hornhaut 2 im betriebsbereiten Zustand in den Bestrahlungskanal 26 hineinragen und die Bestrahlung des Zielgewebes im Bestrahlungskanal 26 bzw. innerhalb des Ringkörpers 20 erfolgen. Im proximalen Teil des Ringkörpers (z.B. an der Stirnfläche 25 des Ringkörpers) sollte daher zumindest jedenfalls kein Formkörper mit Saugfunktion, der von dem Bestrahlungslicht durchstrahlt wird, angebracht sein. Eine tatsächliche Austrittsöffnung existiert dann während der Behandlung also nicht, sondern nur im technischen Sinne, wenn die Vorrichtung nicht am Auge befestigt ist.

Die innere Auflagekante 27 bzw. innere Auflagefläche 28 hat einen Radius von mindestens 3 mm, vorzugsweise von mindestens 4 mm und idealerweise zwischen 5 mm und 6 mm, und in einer bestimmten Ausführungsform 6 mm oder mehr, um die Längsachse 3. Die Ansaugfläche 29 des Saugringes wird durch die Fläche (Ringfläche) zwischen äußerer Auflagefläche 30 und innerer Auflagefläche 28 bestimmt, wobei der Radius der äußeren Auflagekante 31 (bzw. Auflagefläche) mindestens einen halben mm, vorzugsweise über einen mm und idealerweise über 2 mm oder sogar 3 mm, größer sein soll als der Radius der inneren Auflagekante 27 gemessen von der Längsachse 3 der Vorrichtung aus. So kann z.B. die innere Auflagekante 27 (Auflagefläche) einen Durchmesser von 12,0 mm oder 12,5 mm und die äußere Auflagekante 31 (Auflagefläche) einen Durchmesser von 18,0 mm oder 18,5 mm haben.

In einer besonderen Ausführungsform gibt es nur eine der beiden Auflagekanten 24 (Auflageflächen 28, 30), sodass keine spezielle Saugfläche 29 definiert ist. Dabei ist der Saugring 21 nicht als Saugring im eigentlichen Sinne ausgeführt. Unabhängig davon gilt für die gesamte Offenbarung der Begriff Saugring auch für diese Ausführungsform. Dabei erfolgt die Befestigung am Auge nur durch Ausüben eines manuellen Druckes auf die Vorrichtung in Richtung der Längsachse zur Auflagekante hin.

In einer weiteren, in Fig. 2 gezeigten Ausführungsform ist die innere Auflagekante 27 (innere Auflagefläche 28) gegenüber der äußeren Auflagekante 31 (äußere Auflagefläche 30) in Richtung der Längsachse 3 versetzt, um besser an die Krümmung des Auges 1 bzw. der Hornhaut 2 bzw. der Sklera 9 angepasst zu sein. Diese Versetzung sollte möglichst größer als 1 mm und weniger als 3 mm sein, z.B. 1,5 mm, 2 mm oder 2,5 mm. In einer besonderen Ausführungsform ist die Versetzung zumindest 0,5 mm. Die Auflageflächen 28, 30 müssen nicht normal zur Längsachse 3 ausgerichtet sein, sondern können zur noch besseren Anpassung an die Krümmung des Auges 1 bzw. der Hornhaut 2 zusätzlich auch einen Winkel mit einer Ebene normal zur Längsachse 3 einschließen.

Die Aufnahme 22 in Fig. 2 für die Lichtquelle 23 kann einfach als Absatz (Anschlagsbegrenzung, Bereich mit größerem Innendurchmesser als darunter befindlicher Bereich des Ringkörpers 20 (Bestrahlungskanal 26)) im Inneren des Ringkörpers 20 ausgebildet sein: der innere, freie Durchmesser des Ringkörpers ist auf der Höhe der Lichtquelle 23 größer als im Bestrahlungskanal 26. Die Lichtquelle 23 kann damit einfach auf diese als Absatz ausgeführte Aufnahme 22 aufgelegt werden, wodurch die Position der Lichtquelle 23 festgelegt ist. Das gleiche gilt sinngemäß bei Vorhandensein eines Gehäuses, das in die Aufnahme des Ringkörpers eingebracht werden kann, wenn die Lichtquelle sich innerhalb des Gehäuses befindet und auch beim Vorhandensein einer Gehäusehülle, in die das Gehäuse eingesetzt wird, und die dann in die Aufnahme des Ringkörpers eingesetzt wird. In diesen Fällen gilt: Im distalen Teil des Gehäuses (oberhalb der Anschlagsbegrenzung) besitzt das Gehäuse einen Außendurchmesser, der größer als der (jeder) proximale Außendurchmesser (unterhalb der Anschlagsbegrenzung) ist. Im distalen Teil der Gehäusehülle (oberhalb der Anschlagsbegrenzung) ist der (ein) Innendurchmesser größer als der Innendurchmesser des proximalen Teils (unterhalb der Anschlagbegrenzung) der Gehäusehülle. Das gleiche gilt für den Außendurchmesser der Gehäusehülle. Der Außendurchmesser des Gehäuses und der Gehäusehülle liegt zumindest abschnittsweise entlang der Längsachse bevorzugt zwischen 2 mm und 20 mm und in einer besonderen Ausführungsform zwischen 6 mm und 16 mm, wobei die Wandstärke der Gehäusehülle (Unterschied zwischen Außendurchmesser und Innendurchmesser) vorzugsweise zwischen 0,1 mm und 2 mm beträgt. Die genannten Durchmesser werden senkrecht zur Längsachse gemessen. Zur Richtungscharakterisierung entlang der Längsachse wird folgende Konvention verwendet: In der Anatomie des Menschen gibt es die Begriffe distal und proximal. Distal bedeutet vom Körper weg und proximal bedeutet zum Körper hin. So ist zum Beispiel die Hand distal vom Ellenbogengelenk und die Schulter proximal vom Unterarm. Da die gegenständliche Vorrichtung dazu vorgesehen ist, am Körper des Menschen (am Auge) angewandt zu werden, besitzt die Vorrichtung einen Bereich, der mit dem Körper bei der Anwendung der Vorrichtung in Kontakt kommt. Dieser Bereich der Vorrichtung ist jenes Ende, gemessen entlang der Längsachse, wo sich die Ansaugfläche, die Auflageflächen und die Auflagekanten befinden und der in der Beschreibung als unten oder folglich in Analogie zur Anatomie, ohne dadurch einen Bezug auf anatomische Strukturen des Körpers zu generieren, als proximal bezeichnet wird. Folglich werden dann jene Strukturen der Vorrichtung die, gemessen entlang der Längsachse, relativ weiter von unten (vom proximalen Bereich) entfernt sind als distal bezeichnet. So ist z.B. die Aufnahme für die Lichtquelle distal vom Saugring.

Die funktionellen Komponenten der Vorrichtung (Elektronik 32, Bestrahlungs- bzw. Lichtquelle 23, Batterie 33, etc.) sind vorzugsweise entlang der Richtung der Längsachse 3 angeordnet, wobei diese nicht notwendigerweise entlang der Längsachse 3 angebracht sein müssen, sondern sich auch in einem geeigneten Abstand von der Längsachse 3 innerhalb des Innenraumes (Aufnahme) der Vorrichtung, also etwa innerhalb des Ringkörpers 20 in Fig. 2, befinden können.

In einer weiteren Ausführungsform gemäß Fig. 3 ist der Ringkörper 20 in Verbindung mit der Aufnahme 22 derart ausgeführt, dass die funktionellen Komponenten (Lichtquelle 23, Steuerelektronik 32 für die Lichtquelle, Batterie 33 als Energiequelle sowie ein Schalter 46; alle verbunden durch elektrische Verbindungen 44) in einem separaten Gehäuse 34 untergebracht sind, welches mit dem Ringkörper 20 sowie dem Aufsatz für die Aufnahme 22 nicht fest verbunden ist. Der Aufsatz für die Aufnahme 22 ist in diesem Fall einteilig mit dem Ringkörper 20 ausgebildet und bildet einen Teil des Ringkörpers 20. Die Aufnahme 22 ist also als eine Wechselaufnahme ausgeführt, die das Auswechseln der funktionellen Komponenten bzw. des Gehäuses 34 aus der Vorrichtung bzw. aus dem Aufsatz mit der Aufnahme 22, die mit dem Ringkörper 20 verbunden ist, erlaubt. Insbesondere ist die Aufnahme 22 derart ausgeführt, dass das Gehäuse 34, welches die funktionellen Komponenten enthält, anschlagbegrenzt in die Aufnahme 22 eingebracht und auch wieder aus der Aufnahme 22 entfernt werden kann. Auch das Gehäuse 34 verfügt über eine korrespondierende Anschlagsbegrenzung 35, die, im Zusammenspiel mit der Anschlagsbegrenzung 36 der Aufnahme 22, ein Einschieben des Gehäuses 34 in die Aufnahme 22 über eine definierte Tiefe hinaus verhindert. Dadurch wird ein definierter Abstand 37 der Lichtquelle 23 von der Hornhautoberfläche (bzw. von der Stirnfläche 25 des Ringkörpers 20) und damit eine definierte Bestrahlungsstärke an der Hornhautoberfläche garantiert. Der Abstand der Lichtquelle 23 von der Stirnfläche 25 des Ringkörpers 20 bzw. von der inneren Auflagekante gemessen in Richtung der Längsachse 3 beträgt in der Regel mehr als 1 mm, vorzugsweise mehr als 3 mm und in einer besonderen Ausführungsform mehr als 10 mm, jedoch weniger als 70 mm, vorzugsweise weniger als 50 mm und idealerweise weniger als 30 mm.

Bei der Ausführung der Erfindung nach Fig. 3 kann der Ringkörper 20 mit Aufsatz und Aufnahme 22 aus einem resterilisierbaren, biokompatiblen Material, etwa Stahl oder Titan, ausgeführt werden und das Gehäuse 34, welches die funktionellen Komponenten enthält, z.B. als einfacher Spritzgussteil aus Kunststoff, z.B. aus PMMA, weil das Gehäuse 34 z.B. steril (z.B. Ethylenoxidsterilisation) als Einmalprodukt ausgeführt ist.

Das Gehäuse 34 kann mit oder ohne Boden 38 ausgeführt sein. Der Boden 38 dient dabei als Fenster, das entweder völlig durchlässig für das Bestrahlungslicht ist oder räumlich selektiv über die Bestrahlungsfläche betrachtet abhängig von der Distanz von der Längsachse 3 (radial) oder der Umfangsposition um die Längsachse (zirkulär), also ortsabhängig auf der Fensteroberfläche gemessen senkrecht zur Längsachse (also an verschiedenen Orten an den Durchtrittspunkten des Bestrahlungslichtes durch das Fenster) unterschiedlich durchlässig ist und somit in bestimmten Ausführungsformen als Strahlprofilwandler (siehe unten) wirken kann. Das Fenster 38 kann auch so ausgeführt sein, dass beim Durchtritt des Lichtes durch das Fenster der Austrittswinkel des Bestrahlungslichtes aus dem Fenster vom Eintrittswinkel in das Fenster, gemessen in Bezug auf die Längsachse 3 der Vorrichtung, unterschiedlich ist. Das Bestrahlungslicht also beim Durchtritt durch das Fenster vom oder zum Lot gebrochen wird. Das Fenster kann auch so ausgeführt sein, dass das Ausmaß dieser Lichtbrechung (Winkeländerung) von der Position, also dem Ort auf der Fensteroberfläche, also von der Distanz von der Längsachse 3, abhängt bzw. sogar von der Position auf der Fensterfläche, die sich senkrecht zur Längsachse 3 ausdehnt, abhängt. Die genannten Funktionen des Fensters können auch durch geeignete Komponenten, die oberhalb (distal) oder unterhalb (proximal) des Fensters, gemessen in Richtung der Längsachse, angebracht sind, bewirkt werden, z.B. durch Komponenten die in Form von Einlageelementen (z.B. Blättchen) in die Gehäusehülle.

In einer besonderen Ausführungsform kann an einer anderen Stelle innerhalb oder außerhalb des Gehäuses 34 entlang der Richtung der Längsachse 3, aber in Bestrahlungsrichtung nach der Lichtquelle 23, ein weiteres oder anderes Fenster angebracht sein, dass ebenfalls die oben beschriebenen Eigenschaften aufweisen kann. Das Fenster (die Fläche bzw. Substanz des Fensters) dehnt sich im Wesentlichen senkrecht zur Längsachse 3 aus, wenn es auch entlang dieser Ausdehnung über eine unterschiedliche oder variable Dicke verfügen kann. Die Dicke an jedem Punkt der Ausdehnung (Fläche) des Fensters wird in Richtung der Längsachse 3 gemessen.

Anstelle der eingebauten Batterie 33 kann auch eine externe Strom- bzw. Spannungsquelle verwendet werden. Auch die Batterie 33 kann extern, also nicht in die auf das Auge aufsetzbare Vorrichtung, vorgesehen und mit einem Kabel bzw. Draht mit der Steuerelektronik 32 verbunden sein.

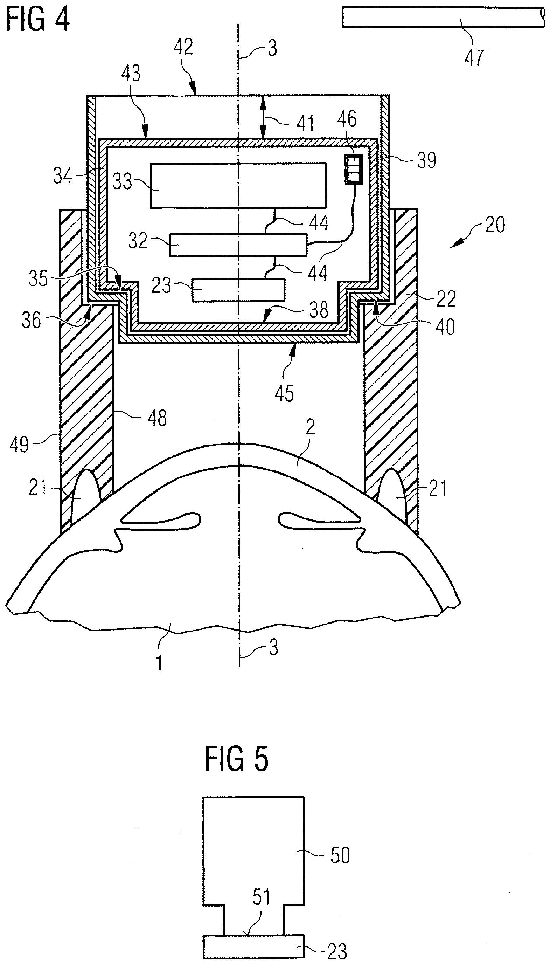

In einer weiteren Ausführungsform gemäß Fig. 4 wird im betriebsbereiten Zustand zusätzlich als Zwischenteil zwischen Gehäuse 34 für die funktionellen Komponenten und der Aufnahme 22 (anschlagbegrenzte Wechselaufnahme) des Aufsatzes bzw. Ringkörpers 20 eine Gehäusehülle 39 angebracht, die selbst wiederum innen eine Anschlagbegrenzung 40 für die anschlagbegrenzte Aufnahme des Gehäuses 34 aufweist und außen eine Anschlagbegrenzung für die Aufnahme der Gehäusehülle 39, die das Gehäuse 34 aufnimmt, in die Aufnahme 22 des Ringkörpers 20 aufweist. Dadurch braucht das Gehäuse 34, das die funktionellen Komponenten (Lichtquelle 23, Steuerelektronik 32, Batterie 33, Schalter 46) enthält, nicht sterilisierbar ausgeführt werden, bzw. nicht steril sein. Die Gehäusehülle 39, die das Gehäuse 34 aufnehmen soll, kann daher als sehr einfacher und kostengünstiger Spritzgussteil aus biokompatiblen mit Etylenoxid oder Gamma-Strahlung sterilisierbarer Kunststoff (z.B. PMMA) als Einmalprodukt ausgeführt werden. Der Außenteil der Vorrichtung, also der Ringkörper 20 (mit Saugring 21, Aufsatz und Aufnahme 22) kann als wiederverwendbares Teil, z.B. für Dampfsterilisation, etwa aus Stahl oder Titan oder einem anderen geeigneten biokompatiblen Material gefertigt werden. Damit die Sterilität der Vorrichtung während der Operation bei dieser Ausführungsform sichergestellt werden kann und eine Übertragung von Keimen vom nicht sterilen Gehäuse 34 auf das Operationsfeld vermieden wird, muss die Höhe der Gehäusehülle 39, gemessen in Richtung der Längsachse 3, größer als jene des Gehäuses 34 sein. Vorzugsweise ist die Höhe der Gehäusehülle 39 im Betriebszustand um mindestens 1 mm größer als die Höhe des Gehäuses 34. Die Höhe des Gehäuses wird gemessen von der Unterseite 38 bis zur Oberkante 43 des Gehäuses in Richtung der Längsachse 3. Die Höhe der Gehäusehülle wird gemessen von der Unterseite 45 bis zur Oberkante 42 der Gehäusehülle in Richtung der Längsachse 3. Idealerweise beträgt diese Höhendifferenz 41 zwischen Gehäuse 34 und Gehäusehülle 39 aber mehr als 2 mm z.B. 5 mm. Mit anderen Worten: Die Oberkante 42 (Oberseite) der Gehäusehülle 39 überragt die Oberkante 43 (Oberseite) des Gehäuses 34 im betriebsbereiten Zustand, also, wenn beide in die dazugehörigen Aufnahmen 22 des Ringkörpers 20 bzw. in die Aufnahme der Gehäusehülle 39 anschlagsbegrenzt eingebracht sind, um mehr als 1 mm, vorzugsweise mehr als 2 mm bzw. idealerweise mehr als 5 mm. Die Oberkante ist jener Punkt oder Kante oder Fläche des Gehäuses 34 bzw. der Gehäusehülle 39, der im betriebsbereiten Zustand am weitesten distal vom Körper (Auge 1, Hornhaut 2) entfernt ist. Jener Punkt oder Kante oder Fläche also, der/die am weitesten vom Saugring 21 entfernt ist, gemessen entlang der Längsachse 3. Proximal (unten, hinten) ist der Saugring 21. Distal (oben, vorne) ist die Aufnahme 22.

In der betriebsbereiten Ausführungsform gemäß Fig. 3 ist der Ringkörper vorzugsweise aus Metall oder Keramik steril (resterilisierbar) und das Gehäuse steril, vorzugsweise als Einmalprodukt ausgeführt.

In der betriebsbereiten Ausführungsform nach Fig. 4 ist vorzugsweise der Ringkörper steril, die Gehäusehülle steril und das Gehäuse unsteril ausgeführt. Dadurch können mögliche Zerstörungen von funktionellen Komponenten bei der Sterilisation vermieden werden.

Die Vorrichtung wird vorzugsweise im betriebsbereiten Zustand so auf dem Auge 1 aufgesetzt, bzw. angebracht, dass die Längsachse 3 der Vorrichtung im Wesentlichen senkrecht zur sagittalen Körperebene ausgerichtet ist, bzw. etwa in Richtung der optischen bzw. anatomischen Achse des Auges 1. Vorzugsweise soll die Längsachse 3 der Vorrichtung etwa mit der Verlaufsrichtung der optischen Achse, bzw. der anatomischen Achse des Auges übereinstimmen, bzw. diese fortsetzen. Der Durchtritt der Längsachse 3 der Vorrichtung durch die vom Saugring 21 umgebene Durchtrittsöffnung (Durchtrittsfläche) 19 für das Bestrahlungslicht (Bestrahlungsfläche, Bestrahlungsöffnung,...) definiert einen Mittelpunkt (Zentrum), um den sich der Saugring 21 konzentrisch anordnet. Dieser Mittelpunkt sollte im betriebsbereiten Zustand vorzugsweise annähernd mit dem Mittelpunkt (Zentrum) der Hornhaut 2, der sich aus dem Durchtrittspunkt der optischen Achse, bzw. der anatomischen Achse durch die Hornhaut 2 ergibt, übereinstimmen. So ist sicher gestellt, dass keine Schädigung der limbalen Stammzellen erfolgt, also der Limbus 10 von der Bestrahlung weitgehend ausgenommen ist.

In verschiedenen Ausführungsformen kann die Längsachse 3 der Vorrichtung an unterschiedlichen Augenachsen, d.h. diese Augenachsen fortsetzend, ausgerichtet sein. So kann in einer bestimmten Ausführungsform die Längsachse 3 der Vorrichtung an der optischen Achse ausgerichtet sein, oder an der anatomischen Achse oder an einer Achse, die zwischen der optischen und der anatomischen Achse liegt.

Auch in dieser Ausführungsform sind die funktionellen Elemente wie z.B. Lichtquelle 23, Elektronik 32, Batterie 33 durch Draht oder Kabel (elektrische Verbindungen 44) miteinander verbunden.

In einer bestimmten Ausführungsform besitzt die Gehäusehülle 39 an der Oberseite (Oberkante 42) eine Öffnung zum Einführen des Gehäuses 34 in die Gehäusehülle 39, vorzugsweise bis zur Anschlagsbegrenzung 40. Die Gehäusehülle 39 oder zumindest das Fenster der Gehäusehülle ist vorzugsweise transparent für das Bestrahlungslicht ausgeführt.

In einer bestimmten Ausführungsform können die Eigenschaften der Unterseite 45 (Fenster) der Gehäusehülle (45) jenen der oben in Fig. 3 beschriebenen für die Unterseite (den Boden/das Fenster 38) des Gehäuses 34 ganz oder teilweise entsprechen und zumindest teilweise als Strahlprofilwandler wirken bzw. ausgeführt sein.

In allen Ausführungsformen mit Wechselaufnahmen werden die auswechselbaren Elemente der Vorrichtung, wie Gehäuse 34 oder Gehäusehülle 39, vorzugsweise in Richtung der Längsachse 3 von oben (distal), das ist das der Hornhaut 2, bzw. dem Saugring 21 entgegengesetzte bzw. abgewandte Ende der Vorrichtung (des Ringkörpers 20), eingeführt. Die Teile der Vorrichtung, die über eine Wechselaufnahme verfügen (Ringkörper 20 mit Aufnahme 22, Gehäusehülle 39) sind nach oben (distal), z.B. an der Oberkante 42, offen ausgeführt.

In einer bestimmten Ausführungsform wird ein Schalter 46 zum Einschalten der Lichtquelle 23 (z.B. UV LED) als mechanischer Schalter ausgeführt. In einer anderen speziellen Ausführungsform wird dieser Schalter 46 berührungsfrei ausgeführt, z.B. indem das Gehäuse 34, das die funktionellen Komponenten enthält, auch einen magnetischen Sensor mit Schalter bzw. Schalterfunktion aufweist, der bei ausreichendem Magnetfeld geeignet ist, den Betriebsstrom für die Lichtquelle einzuschalten bzw. den Beginn der Bestrahlung und in einer weiteren bestimmten Ausführungsform auch das Ende (die Beendigung) der Bestrahlung auszulösen. In einer bestimmten Ausführungsform kann die Bestrahlungszeit auch über einen internen, vorzugsweise elektronischen Timer bzw. Takt- bzw. Zeitgeber definiert bzw. gesteuert werden, indem z.B. nach Einschalten der Bestrahlung durch Auslösen eines magnetischen oder anderen Schalters nach einer bestimmten vorprogrammierten Zeit mittels internen Timers (elektronischer Timer innerhalb der Vorrichtung (Steuerelektronik 32) die Bestrahlung automatisch, d.h. ohne äußeres Zutun, ausschaltet.

In einer besonderen Ausführungsform wird das Ein- und/oder Ausschalten des Bestrahlungsvorganges durch die Vorrichtung mittels eines sterilen Magneten 47 bzw. Magnetstabes, der vom Operateur oder einer Operationsassistenz ausreichend nahe an die betriebsbereite Vorrichtung während der Operation herangebracht wird, ausgelöst. In diesem Falle befindet sich der magnetische Schalter 46 vorzugsweise innerhalb des Gehäuses 34. In diesem Fall ist der Magnet 47 bzw. der Magnetstab Teil der Vorrichtung.

Als betriebsbereit wird jener Zustand der Vorrichtung verstanden, bei dem die jeweiligen Teile der Vorrichtung (je nach Ausführungsform Ringkörper 20 (mit Saugring 21), Aufnahme 22, Gehäuse 34, Gehäusehülle 39, etc.), so zusammengefügt sind und sich in einem solchen Zustand (z.B. Temperatur) befinden, dass nach Einschalten der Vorrichtung (Bestrahlung) diese in ebendiesem Zustand unmittelbar therapeutisch eingesetzt werden kann.

Die auf die Hornhaut 2 übertragende Energie bzw. Strahlungsleistung ist jene Energie bzw. jene Strahlungsleistung die, je nach Ausführungsform, vorzugsweise in einer Entfernung von 1 mm bis 5 mm (z.B. 3 mm) von der inneren Auflagekante 27 bzw. von der Stirnfläche 25 des Ringkörpers 20 oder zwischen Unterseite der Gehäusehülle und 5 mm proximal der Unterseite der Gehäusehülle (= zwischen Gehäusehülle und Stirnfläche des Ringkörpers) jeweils auf der Längsachse 3 und jeweils innerhalb des Bestrahlungskanals 26 gemessen wird.

Der Bestrahlungskanal ist proximal durch die Stirnfläche 25, distal durch die Lichtquelle 23 und seitliche durch die Innenwand 48 des Ringkörpers 20 begrenzt. Dabei kann die Wand des Ringkörpers 20 fenestriert sein, also Ausnehmungen aufweisen.

Für eine konventionelle Bestrahlung mittels UV-A Licht bei Corneal Cross Linking sollte eine Gesamtenergie von 5,4 J/cm$^2$ auf die Hornhaut 2 übertragen werden. Diese Gesamtenergie ergibt sich aus der Multiplikation der auf die Hornhaut 2 übertragenen Leistung, die normalerweise zwischen 3 mW/cm$^2$ und 30 mW/cm$^2$ liegt, mit der Bestrahlungszeit, die demgemäß zwischen 180 s und 1800 s liegt. In einer bestimmten Ausführungsform ist der Timer so eingestellt (ausgeführt), dass diese Bedingungen erfüllt sind. In einer speziellen Ausführungsform ist der interne Timer der Vorrichtung so eingestellt (ausgeführt), dass die Vorrichtung während der Bestrahlung der Hornhaut eine Energie von weniger als 5,4 J/cm$^2$ auf die Hornhaut 2 überträgt. In einer bestimmten Ausführungsform ist der interne Timer der Vorrichtung bei voreingestellter Bestrahlungsleistung zwischen 3 mW/cm$^2$ und 30 mW/cm$^2$ so eingestellt (ausgeführt), dass der Energieübertrag auf die Hornhaut 2 während der durch den Timer begrenzten Behandlungszeit weniger als 5,4 J/cm$^2$, insbesondere weniger als 5 J/cm$^2$ und ganz besonders weniger als 3 J/cm$^2$ beträgt, vorzugsweise weniger als 2,5 J/cm$^2$ und idealerweise zwischen 1,5 J/cm$^2$ bzw. 1,8 J/cm$^2$ bzw. 2,0 J/cm$^2$ und 2,5 J/cm$^2$, z.B. ca. 1,8 J/cm$^2$, ca. 2,1 J/cm$^2$, ca. 2,2 J/cm$^2$ oder ca. 2,3 J/cm$^2$ beträgt. Das bedeutet, dass die Vorrichtung bzw. der Timer (Zeitschalter) die Bestrahlung nach einer Zeit automatisch ausschaltet, wenn gemäß obiger Betrachtungen die gewünschte Bestrahlungsenergie erreicht ist.

Der interne Timer (Zeitschalter) der Vorrichtung schaltet daher z.B. gemäß folgender Tabelle 1 die Bestrahlung der Hornhaut 2, je nach Ausführungsform, nach folgender Bestrahlungszeit aus:

Tabelle 1

| Leistung (mW/cm$^2$) | Bestrahlungszeit (s) | Energie (J/cm$^2$) | Bestrahlungszeit (s) | Energie (J/cm$^2$) | Bestrahlungszeit (s) | Energie (J/cm$^2$) |
|---|---|---|---|---|---|---|
| 3,00 | 720,00 | 2,16 | 780,00 | 2,34 | 600,00 | 1,80 |
| 6,00 | 360,00 | 2,16 | 390,00 | 2,34 | 300,00 | 1,80 |
| 9,00 | 240,00 | 2,16 | 260,00 | 2,34 | 200,00 | 1,80 |

(fortgesetzt)

| Leistung (mW/cm$^2$) | Bestrahlungszeit (s) | Energie (J/cm$^2$) | Bestrahlungszeit (s) | Energie (J/cm$^2$) | Bestrahlungszeit (s) | Energie (J/cm$^2$) |
|---|---|---|---|---|---|---|
| 12,00 | 180,00 | 2,16 | 195,00 | 2,34 | 150,00 | 1,80 |
| 15,00 | 144,00 | 2,16 | 156,00 | 2,34 | 120,00 | 1,80 |
| 18,00 | 120,00 | 2,16 | 130,00 | 2,34 | 100,00 | 1,80 |
| 21,00 | 102,86 | 2,16 | 111,43 | 2,34 | 85,71 | 1,80 |
| 24,00 | 90,00 | 2,16 | 97,50 | 2,34 | 75,00 | 1,80 |
| 27,00 | 80,00 | 2,16 | 86,67 | 2,34 | 66,67 | 1,80 |
| 30,00 | 72,00 | 2,16 | 78,00 | 2,34 | 60,00 | 1,80 |

Die Bestrahlungsleistung in mW/cm$^2$ (bzw. in mW bei Betrachtung der bestrahlten Gesamtfläche) kann dabei während der Bestrahlungszeit konstant sein bzw. um einen bestimmten Wert (oder um einen bestimmten Verlauf - siehe unten) variieren bzw. schwanken. In einer weiteren Ausführungsform kann die Bestrahlungsleistung während der Bestrahlungszeit einem definierten Verlauf folgen, wie z.B. linear zu oder abnehmend, nichtlinear zu-oder abnehmend, periodisch schwankend, nicht konstant linear zu- oder abnehmend (d.h. während der Bestrahlungszeit nimmt die Bestrahlungsleistung zu unterschiedlichen Phasen während dieser Bestrahlungszeit unterschiedlich stark linear oder nicht linear zu oder ab), exponentiell zu- oder abnehmend, etc. oder einer beliebigen Kombination dieser Verlaufsformen.
So könnte zum Beispiel ein nicht konstant abnehmender Bestrahlungsverlauf etwa folgender Gesetzmäßigkeit folgen:

$$\text{Bestrahlungsleistung (t)} = k1*t + k2*t + k3*t + f(t),$$

wobei k1, k2, k3 innerhalb der Bestrahlungszeit oder innerhalb einer bestimmten Zeitphase während der Bestrahlungszeit (wobei Dauer der Zeitphase kleiner gleich Bestrahlungszeit gilt) oder in unterschiedlichen Zeitphasen innerhalb der Bestrahlungszeit unterschiedlich konstant oder Null sein können und f(t) auch linear, multilinear, beliebig oder Null innerhalb der Bestrahlungszeit sein kann oder in unterschiedlichen Zeitphasen jeweils unterschiedlich linear, multilinear, beliebig oder Null sein können.
Eine Ausführungsvariante, bei der die übertragene Gesamtenergie ca. 5,4 J bzw. 5,4 J/cm$^2$ beträgt und bei der die Bestrahlungsleistung mit der Zeit abnimmt, kann z.B. wie folgt aussehen (Tabelle 2):

Tabelle 2

| Zeit (s) | Leistung (mW/cm2) | Energie (mJ/cm2) |
|---|---|---|
| 15,00 | 17,80 | 277,50 |
| 30,00 | 16,70 | 536,25 |
| 45,00 | 16,30 | 783,75 |
| 60,00 | 15,80 | 1024,50 |
| 75,00 | 15,40 | 1258,50 |
| 90,00 | 14,80 | 1485,00 |
| 105,00 | 14,30 | 1703,25 |
| 120,00 | 13,80 | 1914,00 |
| 135,00 | 13,40 | 2118,00 |
| 150,00 | 13,00 | 2316,00 |
| 165,00 | 12,60 | 2508,00 |
| 180,00 | 12,30 | 2694,75 |
| 195,00 | 12,00 | 2877,00 |

(fortgesetzt)

| Zeit (s) | Leistung (mW/cm2) | Energie (mJ/cm2) |
|----------|-------------------|------------------|
| 210,00 | 11,70 | 3054,75 |
| 225,00 | 11,40 | 3228,00 |
| 240,00 | 11,20 | 3397,50 |
| 255,00 | 11,00 | 3564,00 |
| 270,00 | 10,85 | 3727,88 |
| 285,00 | 10,60 | 3888,75 |
| 300,00 | 10,40 | 4046,25 |
| 315,00 | 10,20 | 4200,75 |
| 330,00 | 10,10 | 4353,00 |
| 345,00 | 10,00 | 4503,75 |
| 360,00 | 9,91 | 4653,08 |
| 375,00 | 9,90 | 4801,65 |
| 390,00 | 9,85 | 4949,78 |
| 405,00 | 9,80 | 5097,15 |
| 420,00 | 9,75 | 5243,78 |
| 435,00 | 9,71 | 5389,73 |

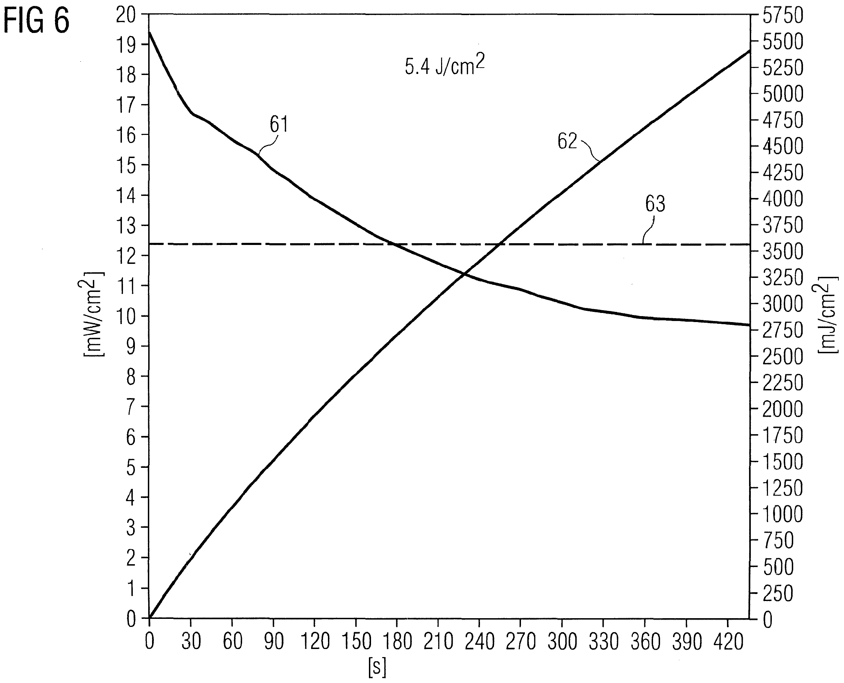

Graphisch ist dies in Fig. 6 dargestellt. Auf der linken senkrechten Achse ist die Leistung in mW/cm$^2$ angegeben, auf der rechten senkrechten Achse die kumulative Energie in mJ/cm$^2$ und auf der waagrechten Achse die Zeit in Sekunden. Dabei ist die Leistung (zeitlicher Verlauf 61 der Bestrahlungsleistung) abnehmend dargestellt, die durch ihren speziellen zeitlichen Verlauf eine kumulierte Energie 62 auf die Hornhaut 2 überträgt, die am Ende der Bestrahlungsdauer eben die kumulierte Gesamtenergie von ca. 5,4 J (bzw. 5,4 J/cm$^2$) auf die Hornhaut 2 übertragen hat. Die mittlere Bestrahlungsleistung 63 ergibt sich aus der kumulierten Gesamtenergie dividiert durch die Bestrahlungsdauer.

In einer anderen Ausführungsvariante, bei der die übertragene Gesamtenergie ca. 2,1 J bzw. 2,1 J/cm$^2$ beträgt und bei der die Bestrahlungsleistung mit der Zeit abnimmt, kann z.B. wie folgt aussehen (Tabelle 3):

Tabelle 3

| Zeit (s) | Leistung (mW/cm2) | Energie (mJ/cm2) |
|----------|-------------------|------------------|
| 15,00 | 17,80 | 277,50 |
| 30,00 | 16,70 | 536,25 |
| 45,00 | 16,30 | 783,75 |
| 60,00 | 15,80 | 1024,50 |
| 75,00 | 15,40 | 1258,50 |
| 90,00 | 14,80 | 1485,00 |
| 105,00 | 14,30 | 1703,25 |
| 120,00 | 13,80 | 1914,00 |
| 135,00 | 13,40 | 2118,00 |

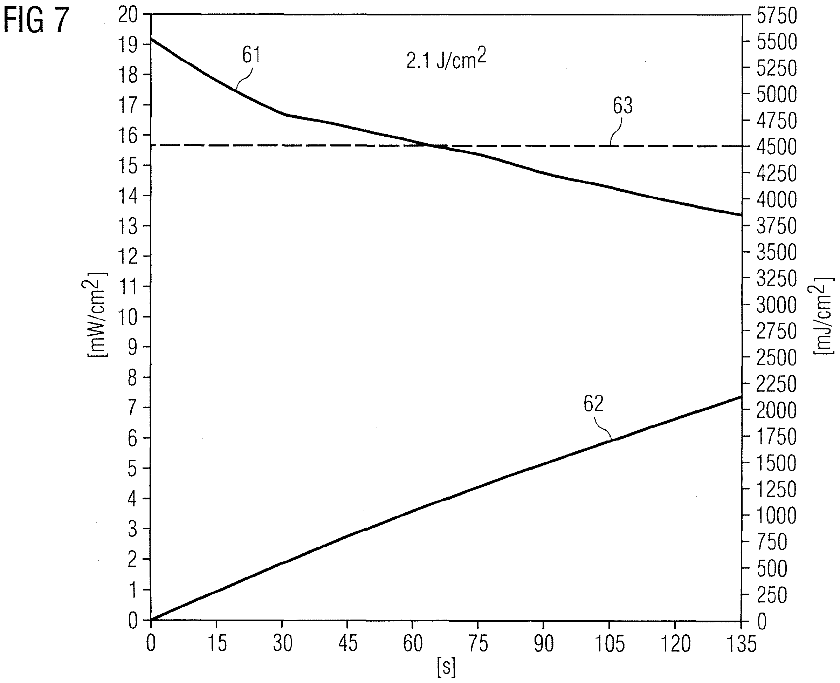

Graphisch ist dies in Fig. 7 dargestellt, wobei Fig. 7 die Verhältnisse von Fig. 6 widerspiegelt, jedoch unter Verwendung einer kumulierten Gesamtenergie von ca. 2,1 J (bzw. 2,1 J/cm$^2$) anstatt 5,4 J (bzw. 5,4 J/cm$^2$). Die auf den einzelnen Achsen aufgetragenen Größen entsprechen jenen in Fig. 6.

In einer bevorzugten Ausführungsform nimmt die Bestrahlungsleistung während der Bestrahlungszeit oder zumindest am Beginn der Bestrahlungszeit mit zunehmender Zeitdauer ab. D.h. die Bestrahlungsleistung ist zu einem späteren Zeitpunkt innerhalb der Bestrahlungszeit geringer als zu einem früheren Zeitpunkt innerhalb der Bestrahlungszeit. In einer speziellen Variante dieser Ausführungsform ist die Abnahme der Bestrahlungsleistung während der Bestrahlungsleistung abnehmend. D.h. in einer späteren Phase der Bestrahlung nimmt die Bestrahlungsleistung mit der Zeit schwächer ab als in einer früheren Phase.

Die übertragene Bestrahlungsenergie (Gesamtenergie) errechnet sich aus dem Integral der zeitabhängigen Bestrahlungsleistung über die Bestrahlungszeit. Die Bestrahlungszeit errechnet sich aus jener Zeitdauer, die benötigt wird, um die notwendige Gesamtenergie, die auf die Hornhaut 2 zu übertragen ist, zu erreichen. Dieser Wert wird in den Timer der Steuerelektronik 32 bei der Fertigung eingestellt (programmiert).

Diese Eigenschaft, dass nämlich die Leistung während der Bestrahlung abnimmt, kann insofern vorteilhaft sein, da sie die technischen Anforderungen an die Kühlung bzw. Wärmeabfuhr der Lichtquelle 23 im betriebsbereiten Zustand bzw. während des Betriebes herabsetzt und dadurch die Kosten der Fertigung senken kann. Auch können dadurch vorteilhaftere Materialien in Bezug auf Transparenz des Gehäuses (Fenster) eingesetzt werden und die Vorrichtung besonders klein gebaut werden, was wieder Vorteile bei der medizinischen Anwendung mit sich bringt.

Vorzugsweise handelt es sich dabei um eine UV-, insbesondere um eine UV-A Lichtquelle 23 zur Bestrahlung der Hornhaut 2. In einer ganz speziellen Ausführungsform handelt es sich bei der Lichtquelle 23 um eine Leuchtdiode, die z.B. im UV-A Bereich beispielsweise Licht in einem Wellenlängenbereich zwischen 350 nm und 370 nm (380 nm), etwa bei 360 nm oder bei 365 nm oder bei 370 nm, abstrahlt. Eine Schwankung bzw. Ungenauigkeit oder der funktionierende Bereich von bis zu 10% um diese Werte kann in bestimmten Ausführungsformen toleriert werden. Diese Lichtquelle 23 kann aber auch in einem völlig anderen Wellenlängenbereich (sichtbar oder unsichtbar) abstrahlen, wenn ein wirksames Agens zur Quervernetzung oder sonstiger Beeinflussung der Kollagenfibrillen oder anderer stabilisierender Elemente der Hornhaut 2 oder diese Elemente selbst in diesem Wellenlängenbereich wirksam sind. Wirksam heißt in diesem Zusammenhang, dass durch die Bestrahlung aus der Lichtquelle 23 mit oder ohne Zuhilfenahme eines in die Hornhaut 2 oder Sklera 9 einzubringenden Agens (z.B. Riboflavin, Hyaluronsäure, etc.) zur Vermittlung des Effektes die Hornhaut 2 strukturell bzw. ultrastrukturell derart verändert wird, dass eine Stabilisierung oder Formveränderung oder Refraktionsänderung oder sonstige Veränderung der Hornhaut 2 oder Sklera 9 erreicht wird. Eine Stabilisierung kann die Hemmung oder Verlangsamung des Fortschreitens einer Erkrankung oder Refraktionsanomalie oder eine zumindest lokale Härtung oder Schrumpfung des Gewebes sein. Die Bestrahlungsquelle (Lichtquelle 23) kann auch aus mehreren einzelnen Bestrahlungsquellen (Lichtquellen) bestehen, die in beliebiger Position zueinander angeordnet sein können und die in besonderen Anwendungen auch unterschiedliche Wellenlängen bzw. Wellenlängenbereiche in der Abstrahlungscharakteristik aufweisen. Die Bestrahlungs- bzw. Lichtquellen können an beliebigen Orten innerhalb der Vorrichtung, auch außerhalb des Gehäuses 34, angebracht sein, vorzugsweise sind alle Lichtquellen jedoch innerhalb des Gehäuses 34 angebracht.

Die Lichtquelle 23 oder Lichtquellen können räumlich oder zeitlich homogen oder inhomogen elektromagnetische Wellen zur zumindest teilweisen Absorption in der Hornhaut 2 oder Sklera 9 abstrahlen. In räumlicher Hinsicht kann die Lichtquelle 23 ein gleichmäßiges Intensitätsprofil über die Abstrahlfläche bzw. über den Abstrahlquerschnitt aufweisen, oder eine bevorzugte Richtcharakteristik, die beispielsweise in eine oder mehrere verschiedene Richtung Intensitätsmaxima aufweisen kann. So kann z.B. eine Richtcharakteristik "keulenartig" mit einem bestimmten Öffnungswinkel (z.B. 80°) sein.

In einer anderen Ausführungsform liegt die Wellenlänge der Lichtquelle 23 zwischen 250 nm und 300 nm, insbesondere zwischen 270 nm und 290 nm, oder aber um oder bei 280 nm. Vorzugsweise ist die Wellenlänge und die Bestrahlungsintensität der Hornhaut 2 so gewählt, dass durch die Bestrahlung kein Gewebsabtrag an der Hornhaut stattfindet. In einer bevorzugten Ausführungsform ist die Wellenlänge und/oder die Bestrahlungsintensität so gewählt, dass ein Gewebsabtrag an der Hornhaut ausgeschlossen ist.

In einer besonderen Ausführungsform kann aber auch die Wellenlänge und die Energie der Lichtquelle 23 (Bestrahlungsquelle) so gewählt werden, dass ein Gewebsabtrag an der Hornhaut 2 erreicht werden kann. In diesem Ausführungsbeispiel sollte die Wellenlänge vorzugsweise zwischen 180 nm und 230 nm liegen. Die Energie sollte zwischen 0,1 und 10 J/cm$^2$ betragen. Die bestrahlte Fläche auf der Hornhaut 2 sollte einen Durchmesser von 12 mm nicht überschreiten, idealerweise sollte sie um 10 mm oder kleiner im Durchmesser sein (z.B. 9,5 mm, 9 mm, 8,5 mm oder 8 mm). Idealerweise ist die bestrahlte Fläche ca. 1 cm$^2$ groß.

Die Bestrahlungs- bzw. Lichtquelle 23 (z.B. LED) ist in einer bestimmten Ausführungsform gemäß Fig. 5 direkt oder indirekt thermisch mit einem festen, metallischen, flüssigen, gasförmigen, salben- oder pastenartigen, fettigen, cremeartigen, amorphen, kristallinen oder sonstigen Kühlkörper 50 bzw. einer Masse innerhalb des Gehäuses 34 verbunden. Der Kühlkörper 50 bzw. die Masse ist geeignet, zumindest einen Teil der von der Lichtquelle 23 erzeugten Wärme aufzunehmen. Mit anderen Worten: Die Temperatur dieses Kühlkörpers 50 nimmt zumindest an einer Stelle oder Fläche an seiner Oberfläche oder im Körperinneren zu. Vorzugsweise ist dieser Kühlkörper 50 mindestens 2 mm breit (gemessen in zumindest einer Dimension senkrecht zur Längsachse 3) und mindestens 2 mm hoch (gemessen in Richtung der Längsachse 3). Idealerweise ist dieser Kühlkörper 50 unmittelbar an der Lichtquelle 23 durch direkten Kontakt an einer Kontaktfläche 51 angebracht, wobei der Abstand zwischen dem Kühlkörper 50 und einer Stelle an der Außenfläche der Lichtquelle 23 möglichst idealerweise Null, mindestens jedoch weniger als 2 mm, vorzugsweise weniger als 0,5 mm beträgt. Die Lichtquelle 23 kann auch mit einer Flüssigkeit in Kontakt sein und/oder von einer solchen innerhalb des Gehäuses 34 umspült sein. Das Bestrahlungslicht durchdringt dann diese Flüssigkeit. Diese Flüssigkeit ist geeignet, Wärme von der Lichtquelle 23 aufzunehmen. Die Flüssigkeit erwärmt sich also während der Bestrahlungszeit.

In einer besonderen Ausführungsform ist die Temperatur der Vorrichtung, insbesondere der Lichtquelle 23 und/oder der Ringkörper 20, in einem betriebsbereiten Zustand weniger als 5°C, vorzugswese weniger als 1 °C und idealerweise möglichst 0°C. In einer weiteren Ausführungsform zeigt die Temperatur der Vorrichtung, insbesondere der Lichtquelle 23 und/oder des Ringkörpers 20, während des Betriebes (= Lichtabstrahlung von der Lichtquelle) einen zeitabhängigen Verlauf. Beispielsweise ist die Vorrichtung so ausgeführt, dass die Temperatur der Vorrichtung, gemessen an der Lichtquelle 21 und/oder dem Ringkörper 20, während des Betriebes (= z.B. während der Zeitdauer die zwischen Einschalten des Bestrahlungsvorganges und dem durch den internen Timer herbeigeführten Ausschalten des Bestrahlungsvorganges) von einer Starttemperatur (z.B. 0°C) im betriebsbereiten Zustand (Startzustard, vordem Einschalten oder zum Zeitpunkt des Einschaltens der Vorrichtung) auf höhere Temperaturwerte (z.B. über 0°C, vorzugsweise über 1°C und insbesondere über 5C) ansteigt.

Die Lichtquelle 23 kann aber auch in einem anderen UV-Bereich, im sichtbaren Lichtbereich oder im Infrarotbereich strahlen.

Als primäre Lichtquellen können auch Festkörperlaser (Neodym oder Titan Laser - z.B. Nd:YAG, Nd:Glas, Nd:YLF, Ti:Sa) eingesetzt werden. Auch können entspreche Harmonische einer Grundwellenlänge erzeugt werden. Dazu können doppelbrechende Kristalle wie BBO, KDP, KTP oder Li-Niobat verwendet werden.

Die Stromzufuhr zum Betreiben der Lichtquelle 23 kann lokal über eine "eingebaute" (interne Energiequelle) Batterie 33 oder einen Akku oder aber über eine Stromleitung zwischen Lichtquelle und Energiequelle (Batterie, Akku, Netzteil oder Trafo) (externe Energiequelle) erfolgen. "Eingebaut" bedeutet in diesem Zusammenhang, dass die Energiequelle mit der Lichtquelle 23 derart fest verbunden ist, dass sie gemeinsam mit der Lichtquelle 23 in den Einsatz des Saugringes (also in den Ringkörper 20) eingesetzt werden kann, auch wenn Teile davon aus dem Ringkörper 20 bzw. der Aufnahme 22 herausragen. Diese Verbindung kann z.B. durch ein gemeinsames Gehäuse 34 erfolgen, das Lichtquelle 23 und Energiequelle 33 nicht nur elektrisch sondern auch mechanisch derart verbindet, dass die Energiequelle 33 durch die mechanische Verbindung mit der Lichtquelle 23 beim Bewegen (z.B. Einsetzen in den Ringkörper 20) derselben automatisch mit dieser mitbewegt werden muss bzw. mitbewegt wird. Mit anderen Worten, die Energiequelle 33 sitzt zumindest während der bestimmungsgemäßen Verwendung fix bzw. fest durch eine mechanische Vorrichtung verbunden auf der Lichtquelle 23 drauf. Dies kann durch ein gemeinsames Gehäuse 34 oder in Form einer gemeinsamen Grundplatte oder sonstiger mechanischer Verbindungselemente zwischen Licht- und Energiequelle 23, 32 realisiert werden.

Durch die, über den am Auge 1 befestigten Ringkörper 20 erreichte Anbringung der Beleuchtungseinrichtung (Vorrichtung) am Auge ist auch jede Unsicherheit und Fehlbeleuchtung des Auges als Folge von willkürlichen und unwillkürlichen Augenbewegungen ausgeschlossen. Zwar beschreibt WO 2011/138031 A1 ein Nachführsystem für einen Beleuchtungsstrahl im Sinne eines scanning spot Strahls zur Sicherstellung einer lokalen Bestrahlungsverteilung und zum Ausschluss von Fehlern durch Augenbewegungen, doch ist dieses System technisch sehr aufwändig und teuer. Es wird demnach durch die erfindungsgemäße Fixierung bzw. der Fixierungsmöglichkeit der Vorrichtung am Auge die Möglichkeit geschaffen, dass die Lichtquelle 23 (Bestrahlungsquelle) selbst mit den Augenbewegungen mitgeführt wird und nicht nur der Strahl aus der Lichtquelle 23 entsprechend abgelenkt wird.

In einer weiteren Ausführungsform der gegenständlichen Erfindung kann zur Homogenisierung des wirksamen Bestrahlungsprofils (Intensitätsprofil der Lichtquelle 23) im Bestrahlungskanal 26 (zwischen Lichtquelle 23 und der Stirnfläche 25 des Ringkörpers 20) mindestens 1 mm proximal der Bestrahlungsquelle (Lichtquelle 23) ein Homogenisator eingebaut werden. Dieser soll durch "Vermischung" des primären Strahlprofils zwischen Lichterzeugung innerhalb der Vorrichtung und Lichtaustritt aus dem Homogenisator vor dem Auftreffen auf der Hornhautoberfläche ein homogenes Intensitätsprofil herstellen. Ein solcher Homogenisator kann beispielsweise in Form eines reflektierenden Hohlzylinders dessen Hohlraum entlang der Zylinderachse (Längsachse 3) oder in einem bestimmten Winkel zur der Bestrahlungsrichtung ausgerichtet ist, ausgeführt sein. Ein (solcher) Homogenisator kann auch konzentrisch oder geneigt oder parallel versetzt oder in einer Kombination dieser Anordnungen zur Bestrahlungsrichtung (welche z.B. durch die Längsachse 3 zwischen Lichtquelle 23 und Stirnfläche 25 des Ringkörpers 20 festgelegt sein kann) angeordnet sein. Die Reflektorfläche des Homogenisators im Inneren kann glatt oder rau oder uneben sein. Sie kann Erhabenheiten oder Vorsprünge aufweisen, die reflektieren oder nicht. Die Reflektorfläche kann auch spiralförmige oder kreisförmige - symmetrische oder unsymmetrische Oberflächengestaltung haben. Besonders einfach kann ein Homogenisator verwirklicht werden, wenn der Bestrahlungskanal 26 durch die Innenwand 48 des Ringkörpers 20 gebildet wird (siehe Fig. 4), weil dann die Innenwand 48 einfach als Reflektorfläche entsprechend ausgebildet werden kann.

In einer weiteren Ausführungsform kann das homogenisierte (Homogenisator) oder nicht homogenisierte Strahlungsprofil (Intensitätsprofil) der Lichtquelle 23 durch eine geeignete Vorrichtung, die im betriebsbereiten Zustand zwischen der Lichtquelle 23 und dem zu bestrahlenden Gewebe (z.B. Hornhaut 2) bzw. in einem Abstand von mindestens 1 mm von der Lichtquelle entfernt (proximal) oder mindestens 1 mm von der Stirnfläche 25 entfernt (distal) innerhalb des Bestrahlungskanals 26 angebracht ist, in ein Strahlungs- bzw. Intensitätsprofil bestimmter Charakteristik umgewandelt werden. Diese Vorrichtung (Strahlprofilwandler) kann bei Vorhandensein eines Homogenisators vor oder nach dem Homogenisator angebracht sein. Bei Nichtvorhandensein eines Homogenisators ist der Strahlprofilwandler zwischen Lichtquelle 23 in einem Abstand von mindestens 1 mm von der Stirnfläche 25 und/oder mindestens 1 mm von der Bestrahlungsquelle innerhalb des Bestrahlungskanals 26 (also zwischen Lichtquelle und Zielgewebe) angebracht. Der Strahlprofilwandler kann mit dem Ringkörper 20 direkt oder indirekt mechanisch verbunden sein oder auch, wie bereits oben ausgeführt, im Gehäuse 34 angeordnet sein, am Fenster (Boden 38, Unterseite) des Gehäuses 34 angebracht sein oder dieses ersetzen oder am Fenster (Unterseite 45) der Gehäusehülle 39 angebracht sein oder dieses ersetzen. Er kann in einer bestimmten Ausführungsform fest mit dem Ringkörper 20 verbunden sein oder austausch- oder auswechselbar sein - das heißt eingesetzt oder entfernt werden. Dies kann z.B. dazu dienen, die unterschiedliche Energiedichte des Lichtes beim Auftreten auf einem zur Bestrahlungsrichtung gekrümmten oder geneigten Zielgewebe (Hornhaut 2) in Joule pro Flächeneinheit oder Watt pro Flächeneinheit auszugleichen. Eine derartige Vorrichtung zur Modifikation des Strahlprofiles (Strahlprofilwandler) kann z.B. aus einem Einschub oder zumindest partiellem optischen Hindernis im Beleuchtungsstrahlengang zwischen der Lichtquelle 23 und dem Zielgewebe (bzw. in einem Abstand von mindestens 1 mm von der Stirnfläche 25 und/oder mindestens 1 mm von der Lichtquelle entfernt innerhalb des Bestrahlungskanals 26) bestehen. Diese Vorrichtung (Einschub, Gehäuse, Gehäusehülle, etc.) zur Strahlmodifikation ist dadurch gekennzeichnet, dass sie zumindest über einen Teil der Bestrahlungsfläche (Intensitätsprofil) ein Transmissionshindernis darstellt. Dies kann in einer besonderen Ausführungsform durch ein Blättchen erreicht werden, das im Strahlengang eingebracht wird und aus einem Material besteht, das für die verwendete Wellenlänge ein Absorptionsverhalten zeigt. Die Intensitätsvariation (Strahlmodifikation) über den Bestrahlungsquerschnitt (Bestrahlungsfläche) kann dabei durch Dickenvariation des Blättchens über dem Beleuchtungsquerschnitt erreicht werden. Dabei ist die Dickenvariation gemessen in Richtung der Längsachse des Blättchens über den Beleuchtungsquerschnitt gemessen senkrecht zur Längsachse ein Abbild der gewünschten Intensitätsvariation. Als Beleuchtungsquerschnitt gilt jede Querschnittsfläche innerhalb des Bestrahlungskanals proximal der Beleuchtungsquelle (also zwischen Beleuchtungsquelle und Stirnfläche bzw. Auflagekante) die sich senkrecht zur Längsachse ausdehnt und durch die Innenwand des Ringkörpers begrenzt ist. Im Falle eines nicht homogenen Strahlprofils vor dem Auftreffen auf das Blättchen muss die Dickenvariation entsprechend mit der Inhomogenität des Primärstrahles vor dem Auftreten auf das Blättchen zur Erzeugung des gewünschten Endintensitätsprofiles beim Auftreffen auf das Zielgewebe gewichtet werden. Im Falle eines homogenen Primärstrahles vor Auftreten auf das Blättchen muss etwa zum Ausgleich des effektiven Abfalles der Lichtwirkung auf das periphere Gewebe infolge der Hornhautkrümmung das Blättchen eine Dickenverteilung haben, bei welcher die Dicke vorzugsweise symmetrisch von innen (entsprechend etwa der optischen Achse) nach außen (Lichtstrahlen die auf die periphere Hornhaut auftreffen) abnimmt. Das Ausmaß der Dicke bzw. der Dickenabnahme hängt vom Absorptionsverhalten des Blättchens (z.B. Fenster im Boden 38 oder Fenster in der Unterseite 45), also dem Absorptionskoeffizienten, und dem genauen bzw. ungefähren Krümmungsverlauf der Hornhaut 2 (je nach Genauigkeitsanspruch der Korrektur) nach außen ab. Es kann aber auch die Variation der Hornhautdicke oder jeder beliebige andere mögliche Grund für eine im Grunde beliebige Variation der Blättchendicke zur Variation der Bestrahlungsintensität herangezogen werden, bzw. eine Kombination von Gründen.

Ein derartiger Strahlprofilwandler kann bei geringer Variation der Bauteile für die primäre Lichtquelle 23 (Toleranz der Bauteile) hinsichtlich Strahlcharakteristik der primären Lichtquelle 23 (Photodiode, Leuchtdiode, UV-Lichtquelle, LED, Laser, Laserdiode, etc.) auch als Homogenisator eingesetzt werden.

Zur Veränderung bzw. Begrenzung des Strahldurchmessers des Bestrahlungslichtes kann im betriebsbereiten Zustand innerhalb des Bestrahlungskanals 26 bzw. beim Austritt des Bestrahlungslichtes aus dem Bestrahlungskanal 26 eine Blende oder ein Blendensystem (z.B. im Gehäuse 34 oder in der Gehäusehülle 39 oder am Ringkörper 20) angebracht sein. Eine solche Blende bzw. ein solches Blendensystem besteht aus einem für die Wellenlängen des Bestrahlungslichtes intransparenten Material mit zumindest einer oder mehreren darauf angebrachten transparenten Lichtdurchlässen. Diese Lichtdurchlässe können eine beliebige Form aufweisen, z.B. können sie rund, kreisrund, elliptisch, sternförmig, eckig oder sonstig sein. Die Blenden können als Einlageblättchen zum Einlegen in die Gehäusehülle 39 ausgeführt sein. Die Lichtdurchlässe können Abmessungen (z.B. Durchmesser oder größte Ausdehnung) von 0,01 mm bis 10 mm, gemessen senkrecht zur Längsachse 3, aufweisen. Die Blende kann auch als "inverse Blende" ausgeführt sein, indem zumindest ein intransparentes Areal von einem transparenten zumindest teilweise umschlossen ist.

Bei allen Einbauten (Homogenisator, Strahlprofilwandler) in das Innere des Ringkörpers, die in den Bestrahlungskanal 26 ragen, ist sicherzustellen, dass diese im betriebsbereiten Zustand der Vorrichtung nicht die Hornhaut 2 berühren. Insofern sollten diese Einbauten - von der Lichtquelle 23 in Richtung der Längsachse 5 gesehen - vor dem Ende des Ringkörpers 20 angeordnet sein, insbesondere in einem Abstand mindestens 1 mm von der inneren Auflagekante 27 (siehe Fig. 2) entfernt.

Der Ringkörper 20 (bzw. seine gerade Außenwand 49, siehe Fig. 4) hat im betriebsbereiten Zustand, gemessen in Richtung der Längsachse 3, eine Höhe von höchstens 150 mm, idealerweise von höchstens 70 mm und von mindestens 10 mm. Im Gehäuse 34 ist die Lichtquelle 23 mindestens 1 mm vom Boden 38 entfernt. Die Lichtquelle 23 ist im betriebsbereiten Zustand mindestens 1 mm, höchstens 150 mm, von der inneren Auflagekante 27 des Ringkörpers 20 entfernt. Eine typische Höhe des Ringkörpers 20 ist etwa 30 mm und der gesamten Vorrichtung, einschließlich Gehäuse 34 und Gehäusehülle 39, etwa 60 mm.

Die erfindungsgemäße Vorrichtung zur Bestrahlung des Zielgewebes kann auch eine Elektronik (Steuerelektronik 32) zur Strombegrenzung für die Stromzufuhr der primären Lichtquelle 23 enthalten. Diese Elektronik 32 kann diese Strombegrenzung entweder durch Pulsierung der Lichtquelle 23 mit festgelegter oder variabler Pulsdauer zur Energiesteuerung der primären Lichtquelle oder durch Begrenzung der Stromstärke durch ohmsche oder aktive Bauteile erzielen. Die elektrische Ansteuerung der Lichtquelle 23 kann in einer besonderen Ausführungsform über eine Konstantstromquelle erfolgen, wobei der Strom durch die Bestrahlungsquelle auf Werte um die 100 mA (50 mA bis 200 mA) liegen sollte. Die Pulsung (Pulsdauer oder relative Pulsdauer zur Periodenlänge) des Lichtes oder des Ansteuerstromes der Lichtquelle kann beliebig sein. Vorteilhaft ist eine Pulsung von etwa 1:2 für das Verhältnis Pulsdauer zu Periodendauer. Die Pulsung kann vorzugsweise zwischen 1:1 und 1:5 liegen. Beispielsweise bei 1:1,5, 1:2, 1:3, 1:4, 1:5 oder auch über 1:5. Die Pulsdauer kann im Bereich von Sekunden, ms, ns, ps oder kleiner liegen.

Der Ruhestrom der Elektronik 32 sollte möglichst im Mikroamperebereich liegen, also unter 100 $\mu$A, vorzugsweise unter 10 $\mu$A.

Die Bestrahlungsintensität, Leistung und Energie am Zielgewebe kann sich u.a. nach der Wahl des gewählten Wirkungsagens im Zielgewebe, nach der gewünschten Bestrahlungsdauer, nach der gewünschten 2-dimensionalen Wirkverteilung (Effekt) entlang der Oberfläche des Zielgewebes (z.B. Hornhautoberfläche), nach der Tiefe bzw. Tiefenverteilung im Zielgewebe, nach der Wellenlänge bzw. dem Wellenlängenspektrum der Lichtquelle 23 (primäre Lichtquelle oder beim Auftreffen auf dem Zielgewebe, oder beim Austreten aus der Vorrichtung), etc. richten.

In einer bestimmten Ausführungsform kann die abgestrahlte Leistung der Vorrichtung auf das Zielgewebe, also die Leistung unmittelbar nach der Lichtquelle 23 (z.B. weniger als 1 mm von der Lichtquelle entfernt), zwischen 1 und 30 mW/cm$^2$, vorzugsweise zwischen 3 und 20 mW/cm$^2$ liegen. In weiteren Ausführungsformen kann die abgestrahlte Leistung zwischen 3 und 15 mW/cm$^2$ oder zwischen 3 und 9 mW/cm$^2$ liegen. Die Leistung kann variabel zwischen den angegeben Werten sein oder fix auf einen bestimmten Wert in einem der angegebenen Bereiche eingestellt (ausgeführt) sein. So können bei Ausführungsformen mit fixer Leistungseinstellung z.B. 3, 6, 9, 10, 12, 15, 20, 25 oder 30 mW eingestellt sein. In einer bestimmten Ausführungsform kann die Leistung über 30 mW/cm$^2$ sein. So sind in diesen Fällen abgestrahlte Leistungen von 30, 35, 40, 45 oder 50 mW/cm$^2$ denkbar.

In einer Ausführungsform kann die Stromstärke zur Ansteuerung bzw. zum Betrieb der primären Lichtquelle 23 (z.B. UV-LED) auf unter 700 mA begrenzt sein. In einer weiteren Ausführungsform kann diese Stromstärke auf unter 200 mA begrenzt sein. In weiteren Ausführungsformen enthält die Vorrichtung eine Spannungsquelle zum Betrieb der primären Lichtquelle 23 mit einer Strombegrenzung auf unter 200 mA, unter 150 mA, unter 125 mA oder unter 100 mA.

In einer Ausführungsform enthält die Vorrichtung zur Bestrahlung des Zielgewebes eine Batterie 33 mit einer Spannung von 3V bis 9V (bzw. bis 12 V), vorzugsweise von 6V, als Energiequelle für den Betrieb der primären Lichtquelle 23.

In einer Ausführungsform enthält die Vorrichtung zur Bestrahlung des Zielgewebes eine Batterie 33 mit einer Kapazität unter 3700 mAh. In einer weiteren Ausführungsform ist die Kapazität der Batterie 33 von 50 bis 3700 mAh, vorzugsweise unter 1500 mAh. In einer weiteren Ausführungsform ist die Kapazität der Batterie unter 200 mAh, bzw. unter 150 mAh. In einer besonderen Ausführungsform ist die Kapazität der Batterie um 100 mAh, z.B. 90 mAh, 100mAh, 105 mAh, also etwa zwischen 80 und 120 mAh. In dieser besonderen Ausführungsform kann bei geeigneter Wahl der primären Lichtquelle 23 der Strom für den Betrieb der primären Lichtquelle 23 durch die Begrenzung der Kapazität der Energiequelle erfolgen, sodass sogar bei Fehlfunktion eine Bereitstellung von zu viel Leistung als Sicherheitsmaßnahme verhindert werden kann, da die Batterie 33 selbst nicht genügend Energie zur Verfügung stellen kann. In diesem Fall muss die Vorrichtung zur Bestrahlung des Zielgewebes eine Vorrichtung zum einfachen Wechsel der Batterie 33, vorzugsweise an der Oberseite (vom Zielgewebe bzw. von der Stirnfläche 25 abgewandten Seite) der Vorrichtung, oder seitlich aufweisen. In einer besonderen Ausführungsform ist die Batterie 33 fest in das Gehäuse 34 eingebaut, sodass sie durch den Benutzer der Vorrichtung nicht ausgewechselt werden kann.

In einer weiteren Ausführungsform ist eine Sicherung im Betriebsstromkreis mit der primären Lichtquelle 23 geschaltet. Diese Sicherung soll eine Ansprechzeit zur Unterbrechung des Stromkreises von unter einer Sekunde, idealer Weise unter 0,1 Sekunde haben.

In einer weiteren Ausführungsform ist ein Timer zum Abschalten der Vorrichtung (z.B. Ausschalten des Betriebsstromes, Blockade der Beleuchtung des Zielgewebes, etc.) zwischen 1 und 30 Minuten Betriebszeit oder Bestrahlungszeit geplant. In einer weiteren Ausführungsform kann dieser Timer auf einen bestimmten Zeitpunkt, etwa 15 Minuten oder 12 Minuten oder 10 Minuten oder 9 Minuten oder 6 Minuten oder 3 Minuten voreingestellt sein.

Der Timer kann bei bestimmten Ausführungsformen, z.B. wenn die Bestrahlungsleistung während der Bestrahlung nicht konstant ist, sondern z.B. abnehmend verläuft, bei der Fertigung so eingestellt werden, das die zeitlich kumulierte Gesamtenergie während der Behandlung den gewünschten Wert (z.B. 5,4 J bzw. 5,4 J/cm$^2$), je nach Verlauf der Bestrahlungsleistung erreicht.

Die Wahl der Betriebs- oder Bestrahlungszeit kann z.B. von der gewählten Leistung, Wellenlänge, intrakornealem Agens, Gewebsdicke etc. abhängen.

In einer bestimmten Ausführungsform kann die Vorrichtung mit einem Applanator zur mechanischen Veränderung der Hornhautgeometrie durch Aufdrücken des Applanators auf die Gewebsoberfläche (z.B. Hornhautoberfläche) ausgestattet sein. Dieser Applanator kann aus für die gewählte Wellenlänge der Lichtquelle 23 transparentem Material (z.B. Quarzglas) oder absorbierendem Material (z.B. PMMA) ausgeführt sein. Der Applanator kann zum Gewebe (also zur Stirnfläche 25 des Ringkörpers 20 hin) eine beliebige Oberflächengeometrie aufweisen. Die Oberflächengeometrie kann auch plan, konvex oder konkav sein. Durch die geeignete Wahl der Oberflächengeometrie kann in einer Ausführungsform die inhomogene Strahlgeometrie (Intensitätsverteilung) oder der Einfluss der peripher zum Strahl gekrümmten Hornhaut 2 ausgeglichen werden, indem ein konvexer Applanator (z.B. zentral vorspringend bzw. konvex) verwendet wird.

Der Applanator kann auch mit einer Vorrichtung zur Strahlmodifikation verbunden werden. So kann z.B. der Applanator zum Zielgewebe hin (also zur Stirnfläche 25 des Ringkörpers 20 hin) plan orthogonal zur Bestrahlungsrichtung und vom Zielgewebe abgewandt in Richtung primärer Lichtquelle 23 konvex mit einem absorbierenden Material für die gewählte Bestrahlungswellenlänge ausgeführt sein. Die Applanationsfläche, bzw. der Applanator kann dabei aus einem für die gewählte Lichtwellenlänge transparenten oder zumindest teilweise absorbierenden Material ausgeführt sein. Bei Verwendung eines Applanators nimmt man bewusst in Kauf, dass die Hornhaut 2 im bestrahlten Bereich vom Applanator berührt wird, jedoch ist dabei die Belastung der Hornhaut 2 im Vergleich zu einer Befestigung der Bestrahlungsvorrichtung im bestrahlten Bereich geringer.

Der Saugring 21 besteht aus zwei konzentrischen Auflageflächen, etwa der inneren Auflagefläche 28 und der äußeren Auflagefläche 30, oder Auflagekanten auf das Zielgewebe, die durch einen partiellen Hohlraum voneinander getrennt sind und den Unterdruck zum Ansaugen der Vorrichtung am Zielgewebe aufnehmen bzw. aufbauen kann.

Der Ringkörper 20 kann aus einem beliebigen, vorzugsweise biokompatiblen Material sein. Er kann z.B. aus Kunststoff (z.B. PMMA) oder Metall (Stahl, Titan, etc.) gefertigt sein. Der Ringkörper 20 kann ein magnetisches Material enthalten oder aus einem solchen bestehen. Die Lichtquelle 23 kann Magnetsensoren (z.B. Hall Sonden) enthalten, die beim Einsetzen in den Ringkörper 20 das magnetische Material detektieren und z.B. als Einschalter für die Lichtquelle 23 verwendet werden können. Auch andere Sensorsysteme sind denkbar.

Der Ringkörper 20, etwa der Saugring 21, oder die Gehäusehülle 39 kann mit einer Vorrichtung ausgestattet sein, die eine Benetzung der Hornhaut 2 mittels Wasser bzw. wässrigen Lösungen während der Behandlung ermöglicht. So kann z.B. die Gehäusehülle 39 bis zu einem bestimmten Ausmaß (z.B. ca. 1 ml oder weniger oder auch mehr, je nach Vorrichtung) an der Fensterseite (am Boden 45) eine oder mehrere Perforationen aufweisen, durch welche das Wasser oder die wässrige Lösung zur Benetzung der Hornhaut 2 langsam über die Behandlungszeit auf die Hornhautoberfläche dringt. Die Öffnungen im Fenster (am Boden 45) der Gehäusehülle 39 sollen dabei möglichst kleiner als 1 mm, vorzugsweise kleiner als 0,5 mm sein.

Technisch gesehen, stellt im betriebsbereiten Zustand jede Seitenwand der Gehäusehülle 39 und des Gehäuses 34, sofern sie distal (oben) über den eigentlichen Ringkörper 20 (Aufnahme) hinausragt, eine Fortsetzung des Ringkörpers 20 dar und kann bei Bedarf zum Ringkörper gezählt bzw. mit ihm gleichgesetzt werden.

Alle Elemente aller Ausführungsformen sind miteinander zu weiteren, neuen Ausführungsformen kombinierbar.

BEZUGSZEICHENLISTE

| | |
|---|---|
| 1 | Auge |
| 2 | Hornhaut |
| 3 | Längsachse (Symmetrieachse) der Vorrichtung, Symmetrieachse des Auges |
| 4 | vordere Augenkammer (Vorderkammer) |
| 5 | hintere Augenkammer (Hinterkammer) |
| 6 | Iris |
| 7 | Pupille |
| 8 | Netzhaut |
| 9 | Sklera |
| 10 | Limbus |
| 11 | - |
| 12 | - |

EP 2 908 788 B1

13 -
14 -
15 -
16 -
17 -
18 -
19   Austrittsöffnung (Durchtrittsöffnung)
20   Ringkörper
21   Saugring
22   Aufnahme
23   Lichtquelle, Bestrahlungsquelle
24   Auflagekante
25   Stirnfläche
26   Bestrahlungskanal
27   Innere Auflagekante
28   Innere Auflagefläche
29   Ansaugfläche
30   Äußere Auflagefläche
31   Äußere Auflagekante
32   Elektronik, Steuerelektronik
33   Batterie (Energiequelle)
34   Gehäuse
35   Anschlagbegrenzung am Gehäuse 34
36   Anschlagbegrenzung an der Aufnahme 22
37   Abstand zwischen Hornhaut 2 bzw. Hornhautoberfläche und der Lichtquelle 23
38   Boden (Unterseite des Gehäuses 34, Fenster)
39   Gehäusehülle
40   Anschlagbegrenzung an der Gehäusehülle 39
41   Höhendifferenz zwischen Gehäuse 34 und Gehäusehülle 39
42   Oberkante der Gehäusehülle
43   Oberkante des Gehäuses
44   Elektrische Verbindung zwischen den funktionellen Elementen wie z.B. Lichtquelle, Elektronik und Batterie
45   Unterseite der Gehäusehülle
46   Schalter
47   Magnet
48   Innenwand des Ringkörpers 20 bzw. des Bestrahlungskanals 26
49   Außenwand des Ringkörpers 20
50   Kühlkörper (Kühlvorrichtung)
51   Kontaktfläche zwischen Kühlkörper 50 und Lichtquelle 23
52 -
53 -
54 -
55 -
56 -
57 -
58 -
59 -
60 -
61   zeitlicher Verlauf der Bestrahlungsleistung
62   zeitlicher Verlauf der kumulierten, an die Hornhaut übertragenen Energie
63   mittlere Bestrahlungsleistung, gemittelt über die Bestrahlungszeit

**Patentansprüche**

1. Vorrichtung zur Bestrahlung der Hornhaut (2) eines Auges (1), die zumindest die folgenden Elemente umfasst:

   - einen Ringkörper (20), der eine konzentrisch um die Längsachse (3) der Vorrichtung ausgeführte Auflagefläche

17

(28, 30) zum Zwecke der Befestigung der Vorrichtung am Auge (1) aufweist,
- einen Bestrahlungskanal (26) zur Bestrahlung der Hornhaut (2), der sich innerhalb des Ringkörpers (20) befindet,
- wobei die Auflagefläche (28, 30) zur Befestigung der Vorrichtung außerhalb der Austrittsöffnung (19) für das Licht aus dem Bestrahlungskanal (26) angeordnet ist,
- sowie eine Lichtquelle (23), die im betriebsbereiten Zustand der Vorrichtung zum Abgeben von Licht in den Bestrahlungskanal (26) innerhalb des Ringkörpers (20) angebracht ist, **dadurch gekennzeichnet, dass** der Bestrahlungskanal (26) durch die Innenwand (48) des Ringkörpers (20) gebildet wird, wodurch ein Homogenisator als Reflektorfläche der Innenwand (48) des Ringkörpers (20) ausgebildet ist, und dass der Homogenisator als reflektierender Hohlzylinder ausgebildet ist.

2. Vorrichtung nach Anspruch 1, **dadurch gekennzeichnet, dass** der Ringkörper (20) eine Aufnahme (22) für eine Lichtquelle (23) aufweist, die als separater Teil gefertigt ist und fest oder lösbar mit dem Ringkörper (20) verbunden ist.

3. Vorrichtung nach Anspruch 2, **dadurch gekennzeichnet, dass** die Aufnahme (22) als Wechselaufnahme ausgeführt ist, die das Auswechseln der funktionellen Komponenten wie z.B. Lichtquelle (23), Steuerelektronik (32) für die Lichtquelle (23), sowie der Batterie (33) als Energiequelle, aus der Aufnahme (22) erlaubt.

4. Vorrichtung nach einem der Ansprüche 1 bis 3, **dadurch gekennzeichnet, dass** die Energiequelle (33) in Form einer Batterie innerhalb des Ringkörpers (20) angebracht ist.

5. Vorrichtung nach einem der Ansprüche 1 bis 3, **dadurch gekennzeichnet, dass** die Energiequelle (33) in Form einer externen Strom- oder Spannungsquelle außerhalb der Vorrichtung angebracht ist.

6. Vorrichtung nach einem der Ansprüche 1 bis 5, **dadurch gekennzeichnet, dass** die Lichtquelle (23) eine UV-Lichtquelle, insbesondere eine UV-A Lichtquelle ist.

7. Vorrichtung nach einem der Ansprüche 1 bis 6, **dadurch gekennzeichnet, dass** die Lichtquelle (23) eine Leuchtdiode ist, die Licht im UV-A Bereich in einem Wellenlängenbereich zwischen 350 nm und 380 nm, insbesondere bei etwa 360 nm oder bei 365 nm oder bei 370 nm, abstrahlt.

8. Vorrichtung nach einem der Ansprüche 1 bis 7, **dadurch gekennzeichnet, dass** die Wellenlänge des von der Lichtquelle (23) ausgehenden Lichts zwischen 180 nm und 230 nm oder zwischen 250 nm und 300 nm, insbesondere zwischen 270 nm und 290 nm, oder aber um oder bei 280 nm liegt.

9. Vorrichtung nach einem der Ansprüche 1 bis 8, **dadurch gekennzeichnet, dass** die Lichtquelle (23) eine bevorzugte Richtcharakteristik mit einem Intensitätsmaximum in eine Richtung aufweist.

10. Vorrichtung nach einem der Ansprüche 1 bis 9, **dadurch gekennzeichnet, dass** der Bestrahlungskanal (26) proximal durch die Stirnfläche (25) des Ringkörpers (20), distal durch die Lichtquelle (23) und seitlich durch die Innenwand (48) des Ringkörpers (20) begrenzt ist.

11. Vorrichtung nach einem der Ansprüche 1 bis 10, **dadurch gekennzeichnet, dass** die Aufnahme (22) eine Anschlagsbegrenzung (35, 36, 40) aufweist.

12. Vorrichtung nach einem der Ansprüche 1 bis 11, **dadurch gekennzeichnet, dass** die Bestrahlungszeit über einen internen, vorzugsweise elektronischen Zeitgeber gesteuert ist.

13. Vorrichtung nach einem der Ansprüche 1 bis 12, **dadurch gekennzeichnet, dass** die am Ende der Bestrahlungszeit auf die Hornhaut übertragene Gesamtenergie 5,4 J/cm2 beträgt.

14. Vorrichtung nach einem der Ansprüche 1 bis 13, **dadurch gekennzeichnet, dass** die Hornhaut (2) im betriebsbereiten Zustand in den Bestrahlungskanal (26) hineinragen und die Bestrahlung des Zielgewebes im Bestrahlungskanal (26) bzw. innerhalb des Ringkörpers (20) erfolgen kann.

**Claims**

1. A device for irradiating the cornea (2) of an eye (1), comprising at least the following elements:

    - a ring body (20), which has a bearing surface (28, 30) embodied concentrically around the longitudinal axis (3) of the device for the purpose of fastening the device on the eye (1),
    - an irradiation channel (26) for irradiating the cornea (2), which is located inside the ring body (20),
    - wherein the bearing surface (28, 30) for fastening the device is arranged outside the outlet opening (19) for the light from the irradiation channel (26),
    - as well as a light source (23), which is mounted inside the ring body (20) in the ready-to-operate state of the device for emitting light into the irradiation channel (26), **characterized in that** the irradiation channel (26) is formed by the inner wall (48) of the ring body (20), whereby a homogenizer is designed as a reflecting surface of the inner wall (48) of the ring body (20), and **in that** the homogenizer is designed as a reflecting hollow cylinder.

2. The device according to claim 1, **characterized in that** the ring body (20) has a receptacle (22) for a light source (23), which is manufactured as a separate part and is fixedly or detachably connected to the ring body (20).

3. The device according to claim 2, **characterized in that** the receptacle (22) is designed as a changeable receptacle which allows the functional components such as e.g. light source (23), control electronics (32) for the light source (23), as well as the battery (33) as energy source, to be changed from the receptacle (22).

4. The device according to one of claims 1 to 3, **characterized in that** the energy source (33) in the form of a battery is mounted inside the ring body (20).

5. The device according to one of claims 1 to 3, **characterized in that** the energy source (33) in the form of an external current or voltage source is mounted outside the device.

6. The device according to one of claims 1 to 5, **characterized in that** the light source (23) is a UV light source, in particular an UV-A light source.

7. The device according to one of claims 1 to 6, **characterized in that** the light source (23) is a light-emitting diode which emits light in the UV-A range in a wavelength range between 350 nm and 380 nm, in particular at about 360 nm or at 365 nm or at 370 nm.

8. The device according to one of claims 1 to 7, **characterized in that** the wavelength of the light emitted by the light source (23) is between 180 nm and 230 nm or between 250 nm and 300 nm, in particular between 270 nm and 290 nm, or around or at 280 nm.

9. The device according to one of claims 1 to 8, **characterized in that** the light source (23) has a preferred directional characteristic with an intensity maximum in one direction.

10. The device according to one of claims 1 to 9, **characterized in that** the irradiation channel (26) is delimited proximally by the end face (25) of the ring body (20), distally by the light source (23) and laterally by the inner wall (48) of the ring body (20).

11. The device according to one of claims 1 to 10, **characterized in that** the receptacle (22) has a stop-delimitation (35, 36, 40).

12. The device according to one of claims 1 to 11, **characterized in that** the irradiation time is controlled via an internal, preferably electronic, timer.

13. The device according to one of claims 1 to 12, **characterized in that** the total energy transferred to the cornea at the end of the irradiation time is 5.4 J/cm$^2$.

14. The device according to one of claims 1 to 13, **characterized in that** in the ready-to-operate state the cornea (2) can protrude into the irradiation channel (26) and the irradiation of the target tissue can take place in the irradiation channel (26) or within the ring body (20).

## EP 2 908 788 B1

**Revendications**

1. Dispositif pour l'irradiation de la cornée (2) d'un œil (1), comprenant au moins les éléments suivants :

    - un corps annulaire (20) qui présente une surface d'appui (28, 30) concentrique autour de l'axe longitudinal (3) du dispositif pour la fixation du dispositif sur l'œil (1),
    - un conduit d'irradiation (26) pour l'irradiation de la cornée (2), qui se trouve à l'intérieur du corps annulaire (20),
    - la surface d'appui (28, 30) pour la fixation du dispositif étant disposée à l'extérieur de l'ouverture de sortie (19) de la lumière hors du conduit d'irradiation (26),
    - et une source lumineuse (23), qui est disposée, dans l'état prêt à fonctionner du dispositif, à l'intérieur du corps annulaire (20) pour émettre de la lumière dans le conduit d'irradiation (26), **caractérisé en ce que** le conduit d'irradiation (26) est formé par la paroi intérieure (48) du corps annulaire (20), ce qui forme un homogénéisateur servant de surface de réflecteur de la paroi intérieure (48) du corps annulaire (20), et **en ce que** l'homogénéisateur est construit comme un cylindre creux réfléchissant.

2. Dispositif selon la revendication 1, **caractérisé en ce que** le corps annulaire (20) présente un logement (22) pour une source lumineuse (23), qui est fabriquée comme une pièce séparée et assemblée de façon fixe ou amovible au corps annulaire (20).

3. Dispositif selon la revendication 2, **caractérisé en ce que** le logement (22) est réalisé comme un logement interchangeable, permettant d'échanger les composants fonctionnels, par exemple la source lumineuse (23), l'électronique de commande (32) pour la source lumineuse (23) et la batterie (33) servant de source d'énergie, en les sortant du logement (22).

4. Dispositif selon l'une des revendications 1 à 3, **caractérisé en ce que** la source d'énergie (33) est installée à l'intérieur du corps annulaire (20) sous la forme d'une batterie.

5. Dispositif selon l'une des revendications 1 à 3, **caractérisé en ce que** la source d'énergie (33) est installée à l'extérieur du dispositif sous la forme d'une source de courant ou de tension externe.

6. Dispositif selon l'une des revendications 1 à 5, **caractérisé en ce que** la source lumineuse (23) est une source de lumière UV, en particulier une source de lumière UV-A.

7. Dispositif selon l'une des revendications 1 à 6, **caractérisé en ce que** la source lumineuse (23) est une diode électroluminescente qui émet de la lumière dans la bande des UV-A dans une bande de longueurs d'onde comprise entre 350 nm et 380 nm, en particulier à environ 360 nm ou à 365 nm ou à 370 nm.

8. Dispositif selon l'une des revendications 1 à 7, **caractérisé en ce que** la longueur d'ondes de la lumière sortant de la source lumineuse (23) est comprise entre 180 nm et 230 nm ou entre 250 nm et 300 nm, en particulier entre 270 nm et 290 nm, ou se situe vers ou à 280 nm.

9. Dispositif selon l'une des revendications 1 à 8, **caractérisé en ce que** la source lumineuse (23) présente une caractéristique directionnelle privilégiée avec un maximum d'intensité dans une direction.

10. Dispositif selon l'une des revendications 1 à 9, **caractérisé en ce que** le conduit d'irradiation (26) est délimité du côté proximal par la face frontale (25) du corps annulaire (20), du côté distal par la source lumineuse (23) et sur le côté par la paroi intérieure (48) du corps annulaire (20).

11. Dispositif selon l'une des revendications 1 à 10, **caractérisé en ce que** le logement (22) présente une limitation à butée (35, 36, 40).

12. Dispositif selon l'une des revendications 1 à 11, **caractérisé en ce que** le temps d'irradiation est commandé par une minuterie interne, de préférence électronique.

13. Dispositif selon l'une des revendications 1 à 12, **caractérisé en ce que** l'énergie totale transmise à la cornée à la fin du temps d'irradiation est de 5,4 J/cm$^2$.

14. Dispositif selon l'une des revendications 1 à 13, **caractérisé en ce que** dans l'état prêt à fonctionner, la cornée (2) peut dépasser dans le conduit d'irradiation (26) et l'irradiation des tissus visés peut avoir lieu dans le conduit d'irradiation (26) ou à l'intérieur du corps annulaire (20).

# FIG 1

FIG 2

FIG 3

FIG 4

FIG 5

FIG 6

5.4 J/cm$^2$

EP 2 908 788 B1

FIG 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012047307 A1
- EP 1561440 A1
- US 2009209954 A
- WO 2012127330 A1
- WO 2011138031 A1

**In der Beschreibung aufgeführte Nicht-Patentliteratur**

- **A. DAXER et al.** Corneal Crosslinking and Visual Rehabilitation in Keratoconus in One Session Without Epithelial Debridement: New Technique. *CORNEA,* 2010, vol. 29, 1176-1179